# EUROPEAN PATENT SPECIFICATION

(11) **EP 4 169 923 B1**
(45) Date of publication and mention of the grant of the patent: **25.02.2026**
(21) Application number: 21825470.4
(22) Date of filing: 18.06.2021
(51) Int. Cl.: C07D 491/14, A61K 31/407, A61P 25/00, C12R 1/465, C12N 1/20, C12P 17/18

(54) **NOVEL PROLYL FK506 DERIVATIVES HAVING NEURITE GROWTH AND SYNAPSE FORMATION ACTIVITIES AND USES THEREOF**
NEUE PROLYL FK506-DERIVATE MIT NEURITENWACHSTUM UND SYNAPSENBILDUNGSAKTIVITÄT UND VERWENDUNGEN DAVON
NOUVEAUX DÉRIVÉS DE PROLYL FK506 AYANT DES ACTIVITÉS DE CROISSANCE DES NEURITES ET DE FORMATION DE SYNAPSES ET LEURS UTILISATIONS

(30) Priority: 19.06.2020 KR 20200075285
(43) Date of publication of application: 26.04.2023
(73) Proprietor: Molgenbio Co., Ltd., Seoul 08826 (KR)
(72) Inventor: YOON, Yeo-Joon, Seoul 06000 (KR); SONG, Myoung-Chong, Seoul 07030 (KR); JUNG, Jin A, Seoul 01858 (KR); CHEONG, Eunji, Seoul 07983 (KR); KIM, Sangwoo, Seoul 06768 (KR); LEE, Heon Joo, Seoul 06504 (KR)
(74) Representative: Mewburn Ellis LLP
(86) International application number: PCT/KR2021/007681
(87) International publication number: WO 2021/256894

(56) References cited:
- EP-A2- 0 478 235
- WO-A1-2022/139524
- KR-A- 20160 017 267
- KR-A- 20200 072 389
- JUNG JIN A ET AL: "Biosynthesis of Nonimmunosuppressive ProlylFK506 Analogues with Neurite Outgrowth and Synaptogenic Activity", JOURNAL OF NATURAL PRODUCTS, vol. 84, no. 2, 3 February 2021 (2021-02-03), US, pages 195 - 203, XP093098652, ISSN: 0163-3864, DOI: 10.1021/acs.jnatprod.0c00767
- MURTY A. R. C. BULUSU; PETER WALDSTÄTTEN; THOMAS TRICOTET; CHRISTOPHE ROCHAIS; ANDREA STECK; MARKUS BACHER; GERHARD SCHULZ; JOSEF : "New Derivatives of Ascomycin with Modifications in the Amino Acid Region – Synthesis and Biological Activities, and X‐Ray Crystal Structure of 5,6‐Dehydroascomycin", HELVETICA CHIMICA ACTA, VERLAG HELVETICA CHIMICA ACTA., HOBOKEN, USA, vol. 92, no. 5, 28 May 2009 (2009-05-28), Hoboken, USA, pages 839 - 889, XP071270567, ISSN: 0018-019X, DOI: 10.1002/hlca.200800436
- BULUSU, M.A.R.C. WALDSTATTEN, P. SCHULZ, G. GRASSBERGER, M.: "Novel analogues of ascomycin with modifications in the amino acid unit through photochemistry: the synthesis of 5,6-dehydroascomycin, SDZ ASQ 871", TETRAHEDRON LETTERS, ELSEVIER, AMSTERDAM , NL, vol. 45, no. 12, 15 March 2004 (2004-03-15), Amsterdam , NL , pages 2523 - 2526, XP004492772, ISSN: 0040-4039, DOI: 10.1016/j.tetlet.2004.02.013
- PARK, J.W. ; MO, S.J. ; PARK, S.R. ; BAN, Y.H. ; YOO, Y.J. ; YOON, Y.J.: "Liquid chromatography-mass spectrometry characterization of FK506 biosynthetic intermediates in Streptomyces clavuligerus KCTC 10561BP", ANALYTICAL BIOCHEMISTRY, ACADEMIC PRESS, AMSTERDAM, NL, vol. 393, no. 1, 1 October 2009 (2009-10-01), Amsterdam, NL , pages 1 - 7, XP026419315, ISSN: 0003-2697

## Description

### 1. Field of the Invention

The present invention relates to any one compound selected from the group consisting of 9-deoxo-36,37-dihydro-prolyl FK506, 9-deoxo-31-*O*-demethyl-36,37-dihydro-prolyl FK506, 9-deoxo-prolyl FK520, and 9-deoxo-31-O-demethyl-prolyl FK520, or a pharmaceutically acceptable salt thereof; the same compound for use in the prevention or treatment of neuronal diseases; a pharmaceutical composition including the compound or a salt thereof, and an excipient; and a method of producing the novel compound using *Streptomyces kanamyceticus* strain.

### 2. Description of the Related Art

With the rapid growth of the global aging population, the number of patients with neurodegenerative diseases, such as Alzheimer's disease, Parkinson's disease, cerebral infarction, *etc.,* is rapidly increasing. However, for these neurological disorders, fundamental treatment such as repair of damaged nerves or regeneration of neurons has not been carried out, and treatment is being performed only for relieving pain or exacerbation. This is because there have been no drugs capable of providing, beyond the effect of delaying nerve damage, nerve regeneration and recovery effects sufficient for use in the treatment of the neurological disorders. As the number of patients suffering from neurodegenerative diseases is expected to increase continuously, the development of DMTs (disease-modifying treatments) capable of fundamentally suppressing progression of these diseases is becoming more important. Patients who have a disorder caused by nerve damage are greatly restricted in their roles in society and at home, resulting in personal loss and deterioration of their quality of life. Therefore, it is urgent to develop a drug capable of providing a nerve regeneration effect which can be used alone or in combination for various diseases associated with nerve damage.

FK506 binds to FK506-binding protein (FKBP)12 in human cells, and then the FK506-FKBP complex binds to calcineurin (CaN) to inhibit its activity, thereby inhibiting interleukin transcription and exhibiting immunosuppressive activity. It is also known that FK506 binds to FKBP52 (or FKBP51) to exhibit nerve regeneration activity through a mechanism not clearly identified *(*Nat. Chem. Biol. 2015, 11, 33). However, there has been no report about a neuronal regeneration material applicable as a therapeutic agent for neurological damage without side effects due to immunosuppressive activity.

Accordingly, as a result of many efforts, the present inventors have demonstrated neuronal growth-promoting effects of novel compounds, 9-deoxo-36,37-dihydro-prolyl FK506, 9-deoxo-31-O-demethyl-36,37-dihydro-prolyl FK506, 9-deoxo-prolyl FK520, and 9-deoxo-31-O-demethyl-prolyl FK520 (hereinafter collectively referred to as "four kinds of novel compounds"), which are applicable as a main ingredient for a pharmaceutical composition for preventing or treating neuronal diseases, and have developed production processes thereof. Moreover, the present inventors have developed a pharmaceutical composition for preventing or treating neuronal diseases by using the compounds as active ingredients, and found that this composition may be effectively applied as a pharmaceutical composition for preventing or treating neuronal diseases without side effects due to immunosuppressive activity, thereby completing the present invention. In particular, the four kinds of novel compounds of the present invention have a significance in that they have remarkably reduced immunosuppressive activity, as compared with FK506 compounds, and have improved efficacy such as neuronal growth-promoting activity, *etc.*

KR 2016 0017267 A (Intron Biotechnology, Inc.), published on 16 February 2016, discloses non-immunosuppressive FK506 analogues with neuroregenerative activity and the use thereof.

### SUMMARY OF THE INVENTION

An object of the present invention is to provide any one compound selected from the group consisting of 9-deoxo-36,37-dihydro-prolyl FK506, 9-deoxo-31-O-demethyl-36,37-dihydro-prolyl FK506, 9-deoxo-prolyl FK520, and 9-deoxo-31-*O-*demethyl-prolyl FK520, or a pharmaceutically acceptable salt thereof.

Another object of the present invention is to provide the compound above for use in the prevention or treatment of neuronal diseases.

Still another object of the present invention is to provide a pharmaceutical composition comprising the compound above or a salt thereof, and an excipient.

Still another object of the present invention is to provide a method of producing the four kinds of novel compounds, the method including the step of culturing *Streptomyces kanamyceticus.*

### BRIEF DESCRIPTION OF THE DRAWINGS

FIG. 1 shows a result of high-performance liquid chromatography analysis of 9-deoxo-36,37-dihydro-prolyl FK506;
FIG. 2 shows a result of nuclear magnetic resonance (¹H-NMR) analysis of 9-deoxo-36,37-dihydro-prolyl FK506;
FIG. 3 shows a result of nuclear magnetic resonance (¹³C-NMR) analysis of 9-deoxo-36,37-dihydro-prolyl FK506;
FIG. 4 shows a result of nuclear magnetic resonance (COSY-NMR) analysis of 9-deoxo-36,37-dihydro-prolyl FK506;
FIG. 5 shows a result of nuclear magnetic resonance (HSQC-NMR) analysis of 9-deoxo-36,37-dihydro-prolyl FK506;
FIG. 6 shows a result of nuclear magnetic resonance (HMBC-NMR) analysis of 9-deoxo-36,37-dihydro-prolyl FK506;
FIG. 7 shows a result of high-performance liquid chromatography analysis of 9-deoxo-31-O-demethyl-36,37-dihydro-prolyl FK506;
FIG. 8 shows a result of nuclear magnetic resonance (¹H-NMR) analysis of 9-deoxo-31-O-demethyl-36,37-dihydro-prolyl FK506;
FIG. 9 shows a result of nuclear magnetic resonance (¹³C-NMR) analysis of 9-deoxo-31-O-demethyl-36,37-dihydro-prolyl FK506;
FIG. 10 shows a result of nuclear magnetic resonance (COSY-NMR) analysis of 9-deoxo-31-O-demethyl-36,37-dihydro-prolyl FK506;
FIG. 11 shows a result of nuclear magnetic resonance (HSQC-NMR) analysis of 9-deoxo-31-O-demethyl-36,37-dihydro-prolyl FK506;
FIG. 12 shows a result of nuclear magnetic resonance (HMBC-NMR) analysis of 9-deoxo-31-O-demethyl-36,37-dihydro-prolyl FK506;
FIG. 13 shows a result of high-performance liquid chromatography analysis of 9-deoxo-prolyl FK520;
FIG. 14 shows a result of nuclear magnetic resonance (¹H-NMR) analysis of 9-deoxo-prolyl FK520;
FIG. 15 shows a result of nuclear magnetic resonance (¹³C-NMR) analysis of 9-deoxo-prolyl FK520;
FIG. 16 shows a result of nuclear magnetic resonance (COSY-NMR) analysis of 9-deoxo-prolyl FK520;
FIG. 17 shows a result of nuclear magnetic resonance (HSQC-NMR) analysis of 9-deoxo-prolyl FK520;
FIG. 18 shows a result of nuclear magnetic resonance (HMBC-NMR) analysis of 9-deoxo-prolyl FK520;
FIG. 19 shows a result of high-performance liquid chromatography analysis of 9-deoxo-31-O-demethyl-prolyl FK520;
FIG. 20 shows a result of nuclear magnetic resonance (¹H-NMR) analysis of 9-deoxo-31-O-demethyl-prolyl FK520;
FIG. 21 shows a result of nuclear magnetic resonance (¹³C-NMR) analysis of 9-deoxo-31-O-demethyl-prolyl FK520;
FIG. 22 shows a result of nuclear magnetic resonance (COSY-NMR) analysis of 9-deoxo-31-O-demethyl-prolyl FK520;
FIG. 23 shows a result of nuclear magnetic resonance (HSQC-NMR) analysis of 9-deoxo-31-O-demethyl-prolyl FK520;
FIG. 24 shows a result of nuclear magnetic resonance (HMBC-NMR) analysis of 9-deoxo-31-*O*-demethyl-prolyl FK520;
FIG. 25 shows results of examining reduction in the immunosuppressive activity of the four kinds of novel compounds of the present invention;
FIG. 26 shows results of examining neuronal growth-promoting ability of the four kinds of novel compounds of the present invention;
FIG. 27 shows results of examining therapeutic potentials of the four kinds of novel compounds of the present invention on neuronal diseases due to synaptogenic activity (a: hippocampal neurons cultured by exposure to FK506 or four kinds of novel compounds, b: measurement of excitatory synaptic currents in neurons, and c: frequency of excitatory synaptic currents in neurons);
FIG. 28 shows results of examining the ability of the four kinds of novel compounds of the present invention to restore and treat the dopaminergic neuronal circuit in Parkinson's disease animal models (a: change in the density of neuron fibers, b: the number of dopaminergic neuronal cell bodies); and
FIG. 29 shows results of an MTT assay for examining cytotoxicity of the four kinds of novel compounds of the present invention.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present invention is not limited by the specific description described below but is defined by the appended claims.

To achieve the above objects, one aspect of the present invention provides any one compound or a pharmaceutically acceptable salt thereof, the compound selected from the group consisting of 9-deoxo-36,37-dihydro-prolyl FK506 represented by Formula 1, 9-deoxo-31-O-demethyl-36,37-dihydro-prolyl FK506 represented by Formula 2, 9-deoxo-prolyl FK520 represented by Formula 3, and 9-deoxo-31-O-demethyl-prolyl FK520 represented by Formula 4 which are four kinds of novel compounds:

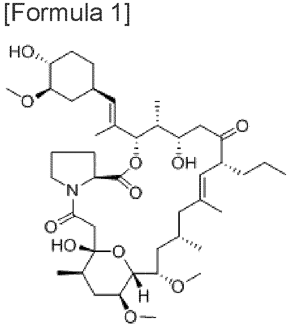

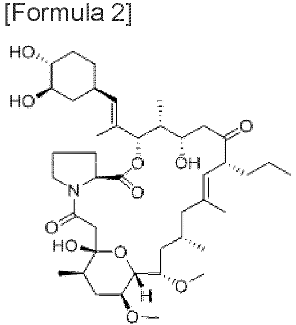

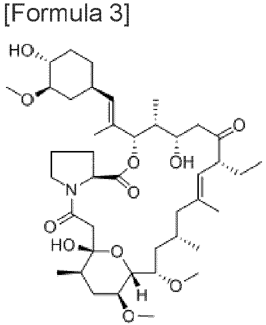

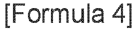

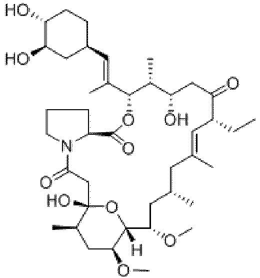

To achieve the above objects, another aspect of the present invention provides a pharmaceutical composition for preventing or treating neuronal diseases, the pharmaceutical composition including any one compound selected from the group consisting of 9-deoxo-36,37-dihydro-prolyl FK506 represented by Formula 1, 9-deoxo-31-O-demethyl-36,37-dihydro-prolyl FK506 represented by Formula 2, 9-deoxo-prolyl FK520 represented by Formula 3, and 9-deoxo-31-O-demethyl-prolyl FK520 represented by Formula 4 which are four kinds of novel compounds, an isomer thereof, or a pharmaceutically acceptable salt thereof as an active ingredient:

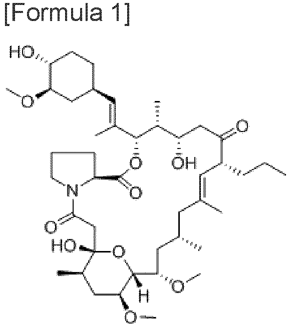

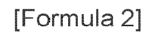

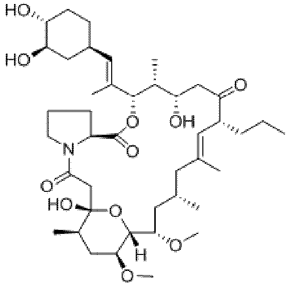

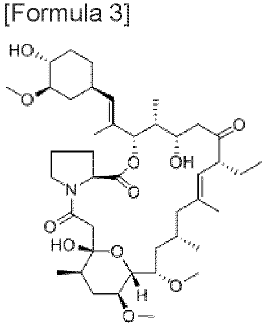

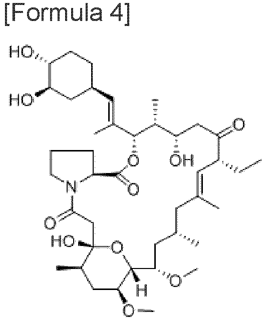

As used herein, the term "FK506, tacrolimus, or fujimycin" refers to a material having an immunosuppressive activity first isolated from a variety of *Streptomyces tsukubaensis,* which is a 23-membered macrocyclic polyketide. Its immunosuppressive activity is stronger than that of cyclosporine, and known to be used to suppress rejection during organ transplantation, particularly, liver transplantation. FK506 may be synthesized by a PKS/NRPS (polyketide synthase/nonribosomal peptide synthetase) complex system, whereas the FK506 derivatives of the present invention may be novel compounds which are produced by novel strains prepared through deletion of biosynthetic genes of *Streptomyces* sp.

As used herein, the term "FK520 or ascomycin" is also an immunosuppressive agent, and is a 23-membered macrolide compound and a C21 ethyl analogue of FK506.

In one specific embodiment of the present invention, the compound of the present invention may include a pharmaceutically acceptable salt.

As used herein, the term "pharmaceutically acceptable salt" refers to any organic or inorganic addition salt whose concentration has effective action because it is relatively non-toxic and harmless to patients and whose side effects do not degrade the beneficial efficacy of a parent compound. For example, the salt may be an acid addition salt formed by a pharmaceutically acceptable free acid. The acid addition salt may be prepared using common methods, for example, by dissolving a compound in an excess aqueous acid solution, and precipitating this salt using a water-miscible organic solvent such as methanol, ethanol, acetone, or acetonitrile. An equimolar compound, and an acid or alcohol in water (e.g., glycol monomethyl ether) are heated, and then the mixture may be dried by evaporation, or the precipitated salt may be filtered by suction. Herein, an organic acid or an inorganic acid may be used as the free acid. The salt may be a pharmaceutically acceptable metal salt prepared using a base.

In another specific embodiment, the compound of the present invention may be in the form of a solvate. The solvate may preferably include a hydrate or an ethanolate.

The pharmaceutical composition of the present invention may be used as a single formulation, and may be used as a complex formulation prepared by additionally including an approved drug which is known to have a therapeutic effect on neuronal diseases. The pharmaceutical composition of the present invention may be formulated using a pharmaceutically acceptable carrier or excipient so as to be provided in a unit dosage form or enclosed into a multi-dose container.

As used herein, the term "pharmaceutically acceptable carrier" may refer to a carrier or diluent that does not impair biological activity or properties of the introduced compound while not irritating an organism. The types of the carrier available in the present disclosure are not particularly limited, and any pharmaceutically acceptable carriers commonly used in the art may be used. Non-limiting examples of the carrier may include co-surfactants exemplified by transcutol, polyethylene glycol, triacetin, and mixtures thereof; surfactants exemplified by cremophor, Tween, Myrj, poloxamer, pluronic, lutrol, imwitor, span, or labrafil alone or in a mixture thereof; oils exemplified by Miglyol, Captex, or ethyl oleate alone or in a mixture thereof; and organic acids exemplified by erythorobic acid, citric acid alone or in a mixture thereof, *etc.* These compounds may be used alone or in a mixture of two or more thereof.

In addition, as needed, other common additives such as an antioxidant, a buffer, and/or a bacteriostatic agent may be added and used, and a diluent, a dispersant, a surfactant, a binder, a lubricant, *etc.* may be further added for formulation into an injectable formulation such as an aqueous solution, a suspension, an emulsion, *etc.,* a pill, a capsule, a granule, a tablet, *etc.*

As used herein, the term "neuronal diseases" collectively refers to diseases of the nervous system, and a specific example thereof may include diseases caused by nerve damage. The neuronal damage diseases may be exemplified by, but are not limited to, neurodegenerative diseases, peripheral nerve injury, traumatic brain injury, and cerebral infarction resulting from cerebrovascular disorders.

For example, the neurodegenerative diseases refer to diseases that cause various symptoms while degenerative changes occur in nerve cells of the central nervous system, and may be, for example, dementia, Alzheimer's disease, Parkinson's disease, progressive supranuclear palsy, multiple system strophy, olivopontocerebellar atrophy (OPCA), Shy-Drager syndrome; striatonigral degeneration, Huntington's disease, amyotrophic lateral sclerosis (ALS), essential tremor, corticobasal ganglionic degeneration, diffuse Lewy body disease, Parkinson-ALS-dementia complex of Guam, or Pick's disease.

For another example, the neuronal damage disease may include epilepsy, stroke, cerebral infarction, ischemic encephalopathy, spinal cord injury disease, peripheral nerve disease, behavioral disorder, developmental disorder, mental retardation, Down syndrome, or schizophrenia, but is limited thereto.

For still another example, the neuronal damage disease may be a disease caused by nerve cell damage or cell death.

As used herein, the term "preventing (or prevention)" means actions by which occurrence of neuronal diseases is restrained or symptoms or conditions are alleviated by administering the compound of the present invention to a subject suspected of having the neuronal disease or a subject having symptoms or conditions associated with the disease.

As used herein, the term "treating (or treatment)" means all of actions by which symptoms of neuronal diseases have taken a turn for the better or been modified favorably by administering the compound of the present invention to a subject suspected of having the neuronal disease.

The four kinds of novel compounds of the present invention may be characterized by the reduced immunosuppressive activity.

In one specific embodiment of the present invention, the immunosuppressive activity of the four kinds of novel compounds was examined by *in vitro* T cell activation assay, and their remarkably reduced immunosuppressive activity, as compared with that of FK506, was observed (Table 7).

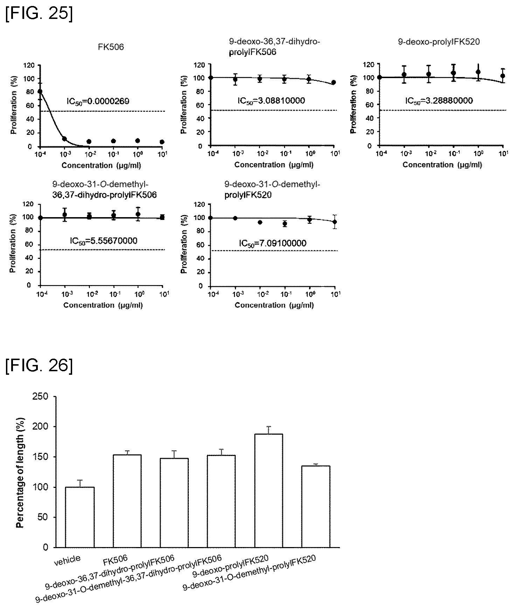

In another specific embodiment of the present invention, the neuronal growth-promoting ability of the four kinds of novel compounds was examined through neurite outgrowth (FIG. 26).

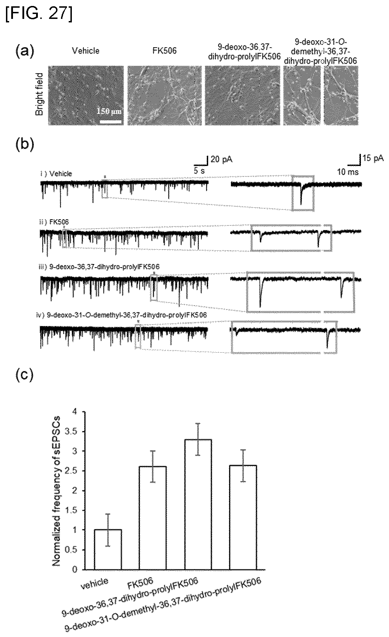

In still another specific embodiment of the present invention, the functional synaptogenic activity of the four kinds of novel compounds was examined, and their increased frequency of excitatory synaptic currents, as compared with that of FK506, was observed, and as a result, the therapeutic effects of the four kinds of novel compounds on the neuronal diseases were confirmed (FIG. 27).

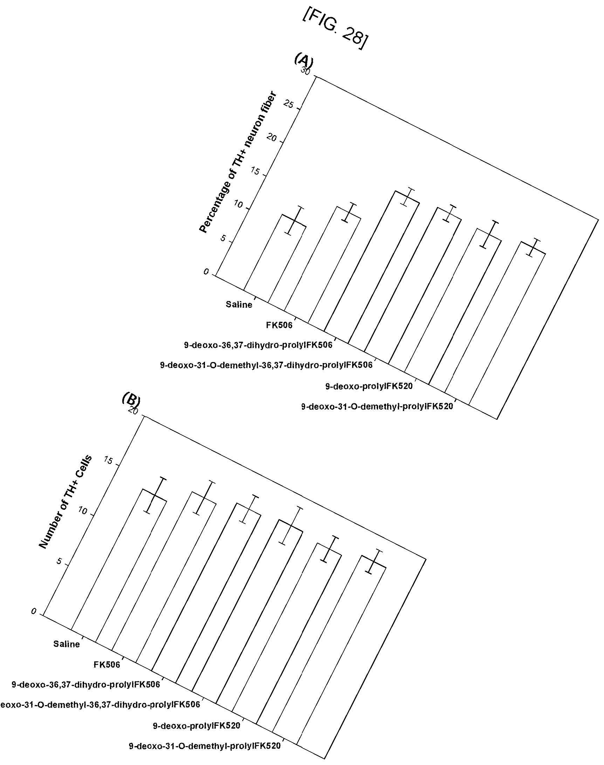

In still another specific embodiment of the present invention, to evaluate *in vivo* neuronal regeneration activity of the four kinds of novel compounds, the degree of recovery of neurons and neuron fibers degenerated by MPTP was examined using, as a disease animal model, a mouse model with Parkinson's disease induced by a neurotoxin 1-methyl-4-phenyl-1,2,3,6-tetrahydropyridine (MPTP), and as a result, the therapeutic effects of the four kinds of novel compounds on the neurodegenerative diseases were confirmed (FIG. 28).

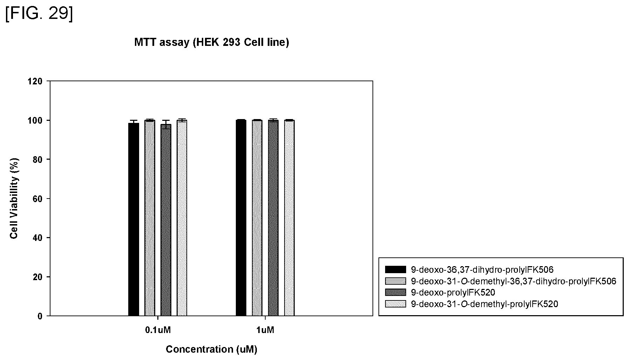

Further, in still another specific embodiment of the present invention, safety was confirmed in a 3-(4,5-dimethylthiazol-2-yl)-2,5-diphenyltetrazolium bromide (MTT) assay (FIG. 29) for cytotoxicity tests of the four kinds of novel compounds, an Ames test for rapid evaluation of genotoxicity, a human eag-related gene (hERG) assay for evaluation of potential effects on cardiac repolarization.

The above results suggest that the four kinds of novel compounds are substances having therapeutic effects and safety on neuronal diseases without side effects due to immunosuppressive activity, and may be usefully applied to the prevention or treatment of neuronal diseases.

Disclosed herein, but not as a claimed invention, is a method of preventing or treating neuronal diseases, the method including the step of administering the pharmaceutical composition to a subject.

As used herein, the terms "neuronal diseases", "preventing", and "treating" are the same as described above.

As used herein, the term "subject" may refer to any animal that has developed or is likely to develop the neuronal disease.

The pharmaceutical composition of the present disclosure may include a pharmaceutically effective amount of one or more selected from the four kinds of novel compounds, or salt thereof. As used herein, the term "pharmaceutically effective amount" means an amount sufficient to treat the disease at a reasonable benefit/risk ratio applicable to any medical treatment. It may be generally administered in an amount of 0.001 mg/kg to 1000 mg/kg, preferably, 0.05 mg/kg to 200 mg/kg, and more preferably, 0.1 mg/kg to 100 mg/kg once or several times a day. However, with respect to the objects of the present disclosure, it is preferred that a specific therapeutically effective amount for a specific patient is differently applied depending on various factors including the type and extent of a response to be achieved, a specific composition including whether or not other formulations are used according to the case, the patient's age, body weight, general health conditions, gender, and diet, administration time, administration route, excretion rate of the composition, duration of treatment, a drug used in combination or concurrently with the specific composition, and similar factors well known in the medical field.

The administration frequency of the pharmaceutical composition of the present disclosure may be administered, but is not particularly limited to, once daily or in a few divided doses.

The administration dose of the pharmaceutical composition of the present disclosure may be 0.001 mg/kg to 1000 mg/kg, specifically 0.05 mg/kg to 200 mg/kg, 0.1 mg/kg to 100 mg/kg, and 0.1 mg/kg to 20 mg/kg, but is not limited thereto.

The pharmaceutical composition of the present disclosure may be administered alone or in combination with other therapeutics, and may be administered together with existing therapeutics sequentially or simultaneously. Single or multiple dosages are possible. It is important to administer the composition in the minimum possible amount sufficient to obtain the maximum effect while minimizing side effects, in view of all the above-described factors, and it may be easily determined by one of ordinary skill in the art.

As used herein, the term "administering" refers to introduction of the pharmaceutical composition of the present disclosure to a patient by any appropriate methods, and the administration of the composition of the present disclosure may be made via various routes of any oral or parenteral route as long as the composition reaches the target tissues.

The administration mode of the pharmaceutical composition according to the present disclosure is not particularly limited, and may follow a method commonly used in the art. For non-limiting examples of the administration mode, the pharmaceutical composition may be administered orally or parenterally. The pharmaceutical composition according to the present disclosure may be prepared into various formulations depending on the desired administration mode.

In one specific embodiment of the present disclosure, it was confirmed that the four kinds of novel compounds showed remarkably reduced immunosuppressive activity, while having superior safety along with the neurite outgrowth and synaptogenic activities and the therapeutic effects on neurodegenerative diseases.

The above results suggest that the four kinds of novel compounds may be continuously used for the treatment of neuronal diseases by remarkably reducing side effects due to immunosuppressive activity.

To achieve the above objects, still another aspect of the present invention provides a method of producing the four kinds of novel compounds, the method including the step of culturing *Streptomyces kanamyceticus,* which is a biological process of producing the four kinds of novel compounds.

Among the four kinds of novel compounds, 9-deoxo-36,37-dihydro-prolyl FK506 or 9-deoxo-prolyl FK520 may be produced by the step of culturing *Streptomyces kanamyceticus* ΔfkbD,tcsD,fkbL (Accession No. KCTC14171BP).

Further, 9-deoxo-31-O-demethyl-36,37-dihydro-prolyl FK506 or 9-deoxo-31-O-demethyl-prolyl FK520 may be produced by the step of culturing *Streptomyces kanamyceticus* ΔfkbD-fkbM,tcsD,fkbL (Accession No. KCTC14170BP).

As used herein, the term "culturing" means growing the microorganism under a properly regulated environment conditions. The culturing process of the present disclosure may be performed under proper medium and culture conditions which are known in the art. The culturing process may be easily adjusted and used by those skilled in the art according to a selected strain. Specifically, the culturing may be batch, continuous, and fed-batch, but is not limited thereto.

The method of producing the four kinds of novel compounds generally uses the culture temperature adopted in a process of culturing *Streptomyces* sp. As a suitable culture temperature for the implementation of the present invention, a culture temperature of 23°C to 30°C may be preferably applied, more preferably, a culture temperature of 25°C to 28°C may be applied.

In addition, in the production method, pH of the culturing process is maintained between 6.5 to 9, and preferably, the culture pH is maintained at 7 to 8.

On the other hand, in the production method, it is important to maintain a high level of dissolved oxygen in the culture medium. When the dissolved oxygen level at the beginning of the culture is 100%, it is important that the dissolved oxygen level is maintained at 30% or more until the end of the culture. In order to achieve this, it is preferable that shaking is generally performed at about 800 rpm to 1,500 rpm.

Extraction of the four kinds of novel compounds produced from the cultured cell bodies in the above production method is achieved through a primary extraction process, a secondary extraction process, and a tertiary extraction process. In the present invention, an organic solvent extraction method is used as the primary extraction process. A solvent applicable to this process may include ethyl acetate, methanol, acetone, *etc.,* but the use of ethyl acetate or methanol is preferred. Silica gel chromatography is used as the secondary extraction process, and as a solvent applicable to this process, methanol and methylene chloride are preferred. Chromatography is used as the tertiary extraction process, and a solvent applicable to this process may include acetonitrile, ammonium acetate buffer, acetic acid, formic acid, *etc.* The use of acetonitrile is preferred. Application of this method facilitates the recovery of four kinds of novel compounds and also increases the yield.

Disclosed herein, but not as a claimed invention, are *Streptomyces kanamyceticus* ΔfkbD-fkbM,tcsD,fkbL (Accession No. KCTC14170BP) and *Streptomyces kanamyceticus* ΔfkbD,tcsD,fkbL (Accession No. KCTC14171BP) which are production strains applicable to the preparation of the four kinds of novel compounds.

The production strains may be recombinant strains, and the recombination may be performed by genetic modification such as transformation.

Disclosed herein, but not as a claimed invention, is a quasi-drug composition for preventing or improving neuronal diseases, the quasi-drug composition including any one compound selected from the group consisting of 9-deoxo-36,37-dihydro-prolyl FK506, 9-deoxo-31-O-demethyl-36,37-dihydro-prolyl FK506, 9-deoxo-prolyl FK520, and 9-deoxo-31-O-demethyl-prolyl FK520, an isomer thereof, or a pharmaceutically acceptable salt thereof as an active ingredient.

The four kinds of compounds, pharmaceutically acceptable salt, neuronal diseases, and preventing are the same as described above.

As used herein, the term "quasi-drug" refers to an article, other than appliances, machinery, or equipment, among articles used for the purpose of diagnosis, treatment, alleviation, handling, or prevention of human or animal diseases, and an article, other than appliances, machinery or equipment, among articles used for the purpose of exerting pharmacological effects upon the structure or functions of human beings or animals.

In the present disclosure, the quasi-drug composition may have effects of preventing or improving neuronal diseases, but is not limited thereto.

The quasi-drug composition of the present disclosure may further include, as needed, a pharmaceutically acceptable carrier, excipient, or diluent, in addition to the above ingredients. The pharmaceutically acceptable carrier, excipient, or diluent is not limited as long as it does not impair the effects of the present disclosure, and it may include, for example, fillers, extenders, binders, wetting agents, disintegrants, surfactants, lubricants, sweeteners, fragrances, preservatives, *etc.*

The "pharmaceutically acceptable carrier" may refer to a carrier, excipient, or diluent that does not impair biological activity or properties of the introduced compound without irritating an organism, and it may be specifically a non-naturally occurring carrier. The types of the carrier applicable in the present disclosure are not particularly limited, and any pharmaceutically acceptable carriers commonly used in the art may be used. Non-limiting examples of the carrier may include saline, sterilized water, Ringer's solution, buffered saline, an albumin injection solution, a dextrose solution, a maltodextrin solution, glycerol, and ethanol, which may be used alone or in a mixture of two or more thereof.

The composition including the pharmaceutically acceptable carrier may be prepared into various formulations suitable for oral or parenteral administration, preferably, formulations for oral administration, but is not limited thereto. For formulation, a commonly used diluent or excipient, such as a filler, an extender, a binder, a humectant, a disintegrant, or a surfactant, *etc.,* may be used. Specifically, solid formulations for oral administration may include tablets, pills, powder, granules, capsules, *etc.,* and these solid formulations may be prepared by mixing the compound with at least one excipient, for example, starch, calcium carbonate, sucrose, lactose, or gelatin. In addition to simple excipients, a lubricant such as magnesium stearate or talc may also be used. Liquid formulations for oral administration may be suspensions, formulations for internal use, emulsions, syrups, *etc.,* and may include various excipients such as humectants, sweeteners, fragrances, and preservatives, in addition to simple diluents commonly used in the art, such as water or liquid paraffin. Formulations for parenteral administration may include sterile aqueous solutions, non-aqueous solvents, suspensions, emulsions, lyophilizates, suppositories, *etc.* The non-aqueous solvents and suspensions may be propylene glycol, polyethylene glycol, vegetable oils such as olive oil, injectable esters such as ethyl oleate, *etc.* Bases for the suppositories may be Witepsol, Macrogol, Tween 61, cacao butter, laurin butter, glycerogelatin, *etc.*

The quasi-drug composition of the present disclosure may be exemplified by, but is not limited to, a disinfectant cleaner, a shower foam, an ointment, a wet tissue, a coating agent, *etc.* The formulation method, dose, usage, components, *etc.* of the quasi-drug may be appropriately selected from common techniques known in the art.

Disclosed herein, but not as a claimed invention, is a food composition for preventing or improving neuronal diseases, the food composition including, as an active ingredient, any one compound selected from the group consisting of 9-deoxo-36,37-dihydro-prolyl FK506, 9-deoxo-31-O-demethyl-36,37-dihydro-prolyl FK506, 9-deoxo-prolyl FK520, and 9-deoxo-31-O-demethyl-prolyl FK520, an isomer thereof, or a salt thereof acceptable for use in food.

The four kinds of compounds, salt acceptable for use in food, neuronal diseases, and preventing are the same as described above.

The food composition of the present disclosure may be daily ingested, and it has advantages of being free from side effects that may occur when taking a drug for a long time, because it contains a natural product as a raw material, unlike general drugs. Therefore, the food composition may be very usefully applied for the purpose of preventing or improving neuronal diseases.

As used herein, the term "improving" means all of actions that alleviate or reduce parameters associated with neuronal diseases, for example, the severity of symptoms by ingestion of the food composition.

As used herein, the term "food" includes meats, sausages, breads, chocolates, candies, snacks, confectionery, pizzas, ramen noodles, other noodles, gums, dairy products including ice creams, various soups, beverages, teas, drinks, alcoholic beverages, and vitamin complexes, health functional foods, health foods, *etc.,* and the food includes all foods in the ordinary acceptation of the term.

The health functional food is the term identical to the food for special health use (FoSHU), and refers to a food having high medical, medicinal effects, which is processed so as to efficiently exhibit the biologically modulating function as well as to supply nutrients.

Here, the "functional" indicates a useful effect for human health, such as regulation of nutrients for the structure and function of the human body, physiological action, *etc.* A health food refers to a food having an active health maintenance or promotion effect, as compared to general foods, and a health supplement food refers to a food for health supplement purposes. In some cases, the terms "health functional food", "health food", and "health supplement food" may be used interchangeably.

Specifically, the health functional food is a food prepared by adding the four kinds of novel compounds to food materials such as beverages, teas, spices, gums, confectionery, *etc.,* or encapsulating, powdering, making into suspension, *etc.* It means that the health functional food has a specific effect on health when ingested, but it has advantages of being free from side effects that may occur when taking a drug for a long time, because it contains a food as a raw material, unlike general drugs.

The food of the present disclosure may be prepared by a method commonly used in the art, and may be prepared by adding raw materials and ingredients which are commonly added in the art.

In addition, the food composition may be prepared into various formulations without limitation as long as the formulation is recognized as food.

Further, the food composition may further include a carrier acceptable for use in food, and the type of the carrier is not particularly limited, and any carrier may be used as long as it is commonly used in the art.

Further, the food composition may further include additional ingredients that are commonly used in food compositions so as to improve smell, taste, vision, *etc.* For example, the food composition may include vitamins A, C, D, E, B1, B2, B6, and B12, niacin, biotin, folate, pantothenic acid, *etc.* Further, the food composition may also include minerals such as Zn, Fe, Ca, Cr, Mg, Mn, Cu, Cr, *etc*.; and amino acids such as lysine, tryptophan, cysteine, valine, *etc.*

Further, the food composition may also include food additives, such as preservatives (potassium sorbate, sodium benzoate, salicylic acid, sodium dehydroacetate, *etc.),* disinfectants (bleaching powder, higher bleaching powder, sodium hypochlorite, *etc.),* antioxidants (butylhydroxyanisole (BHA), butylhydroxytoluene (BHT), *etc.),* coloring agents (tar color, *etc.),* color-developing agents (sodium nitrite, *etc.),* bleaching agents (sodium sulfite), seasonings (monosodium glutamate (MSG), *etc.),* sweeteners (dulcin, cyclemate, saccharin, sodium, *etc.),* flavors (vanillin, lactones, *etc.),* swelling agents (alum, potassium D-bitartrate, *etc.),* fortifiers, emulsifiers, thickeners (adhesive pastes), film-forming agents, gum base agents, antifoaming agents, solvents, improvers, *etc.* The additives may be selected according to the food types and used in an appropriate amount.

Disclosed herein, but not as a claimed invention is, the use of the composition including any one compound selected from the group consisting of 9-deoxo-36,37-dihydro-prolyl FK506, 9-deoxo-31-O-demethyl-36,37-dihydro-prolyl FK506, 9-deoxo-prolyl FK520, and 9-deoxo-31-O-demethyl-prolyl FK520, an isomer thereof, or a pharmaceutically acceptable salt thereof as an active ingredient in preventing, improving, or treating neuronal diseases.

Hereinafter, the present invention will be described in more detail with reference to the following exemplary embodiments. However, these exemplary embodiments are for illustrative purposes only, and the scope of the present invention is not intended to be limited by these exemplary embodiments.

### Example 1: Preparation of 9-deoxo-36,37-dihydro-prolyl FK506

In *Streptomyces kanamyceticus,* which is a strain producing FK506, inactivation of *fkbD, tcsD,* and *fkbL* genes was induced using an in-frame deletion method by double cross-over homologous recombination according to a method described in Ban, Y. H. et al. (J. Nat. Prod. 2013, 76, 1091-1098) to prepare a *Streptomyces kanamyceticus* ΔfkbD,tcsD,fkbL (Accession No. KCTC14171BP), which is a strain producing 9-deoxo-36,37-dihydro-prolyl FK506.

In detail, to prepare a mutant, in which *fkbD, tcsD,* and *fkbL* genes were deleted in the *Streptomyces kanamyceticus* strain producing FK506, each gene was cloned into a pKC1139 vector and transferred to *Escherichia coli* ET12567/pUZ8002, and transformed to FK506-producing *Streptomyces kanamyceticus* strain via conjugation.

The method of preparing the strain may be explained in more detail by construction of an in-frame gene deletion plasmid and construction of a gene deletion strain.

In the construction of the in-frame gene deletion plasmid, an *E. coli-Streptomyces* shuttle vector pKC1139 was used for in-frame gene deletion. The plasmid construction was performed by PCR amplification of left- and right-flanking fragments of the target gene for deletion from fosmid DNA derived from *Streptomyces kanamyceticus.* Primer pairs FkbDLF/FkbDLR and FkbDRF/FkbDRR for the left-flanking fragments and the right-flanking fragments respectively were designed for deletion of the *fkbD* gene. Primer pairs TcsDLF/TcsDLR and TcsDRF/TcsDRR for the left-flanking fragments and the right-flanking fragments respectively were designed for deletion of the *tcsD* gene. Primer pairs FkbLLF/FkbLLR and FkbLRF/FkbLRR for the left-flanking fragments and the right-flanking fragments respectively were designed for deletion of the *fkbL* gene. All the PCR fragments were isolated and digested with HindIII-Xbal or Xbal-EcoRl, and then cloned into the pKC1139 vector. Information regarding the strains, plasmids, and primers used in the present Example are shown in Tables 1 and 2 below.

The plasmids used for the construction of the gene deletion strain are summarized in Table 1. pΔfkbD which is a plasmid to remove C9 hydroxylase was transferred to *E. coli* ET12567/pUZ8002, and then introduced into *Streptomyces kanamyceticus* via conjugation, and the target genes were deleted by homologous recombination. A strain in which a single crossover between the deletion plasmid and the *Streptomyces kanamyceticus* chromosome had occurred was selected by cultivation of an apramycin-resistant transconjugant at 37°C (the non-permissive temperature for the pSG5-based replicon) in the presence of apramycin. Then, the obtained colony was subjected to three rounds of propagation in the absence of selection at 28°C to allow for the second crossover. The two desired double crossover mutant, i.e., ΔfkbD, was selected by the apramycin-sensitive phenotype, then verified by PCR, and selectively confirmed by Southern blot analysis.

To the prepared *fkbD* gene-deleted *Streptomyces kanamyceticus* ΔfkbD, pΔtcsD which is a plasmid for C21 side chain alteration was introduced, and the *tcsD* gene was deleted in the same manner as in the method of deleting the *fkbD* gene. ΔfkbD,tcsD was selected by apramycin-sensitive phenotype, then verified by PCR, and selectively confirmed by Southern blot analysis. Additionally, to the prepared *fkbD-* and tcsD-deleted *Streptomyces kanamyceticus* ΔfkbD,tcsD, pΔfkbL, which is a plasmid for C1 prolyl ring formation, was introduced, and the *fkbL* gene was deleted in the same manner as in the methods of deleting the *fkbD* and *tcsD* genes. ΔfkbD,tcsD,fkbL was selected by apramycin-sensitive phenotype, then verified by PCR.

The prepared *Streptomyces kanamyceticus* ΔfkbD,tcsD,fkbL, which is a *fkbD,tcsD,fkbL* gene-deleted strain, was deposited at the Korean Collection for Type Cultures (KCTC) on April 14, 2020, under Accession No. KCTC14171BP.

**[Table 1]**

| Information regarding strains and plasmids used | |
|---|---|
| Strain/vector | Relevant characteristic |
| Bacterial strains | |
| *Escherichia coli* | |
| *DH5α* | Host for general cloning |
| ET12567/pUZ8002 | Donor strain for intergeneric conjugation between *E. coli* and *Streptomyces* |
| *Streptomyces* | |
| *Streptomyces kanamyceticus* | Wild-type FK506 producing strain |
| ΔfkbD,tcsD,fkbL | Mutant of S. *kanamyceticus* with an in-frame deletion of fkbD,tcsD.fkbL |
| ΔfkbD-fkbM,tcsD,fkbL | Mutant of S. *kanamyceticus* with an in-frame deletion of fkbD-fkbM,tcsD,fkbL |
| Plasmid | |
| pKC1139 | High-copy-number temperature-sensitive *E. coli-Streptomyces* shuttle vector |
| pΔfkbD | Deletion plasmid with in-frame deletion of 51 bp internal fkbD fragment |
| pΔfkbD-fkbM | Deletion plasmid with in-frame deletion of 1100 bp internal fkbDM fragment |
| pΔtcsD | Deletion plasmid with in-frame deletion of 1154 bp internal tcsD fragment |
| pΔfkbL | Deletion plasmid with in-frame deletion of 873 bp internal fkbL fragment |

**[Table 2]**

| Information regarding primers used | | |
|---|---|---|
| Primer | Sequence 5' to 3' (Restriction site underlined) | Restriction enzyme |
| FkbDLF | TATAAAGCTTCGGAGCCCCGGTGGACCT | Hindlll |
| FkbDLR | TTAATCTAGACGTCGCCTCGTCGTCGCT | Xbal |
| FkbDRF | GTAATCTAGAGTCGGCTACTGCCTCTAC | Xbal |
| FkbDRR | GAATGAATTCCGACGAACAGCGGTTCCT | EcoRI |
| FkbD-MLF | TATAAAGCTTCGGAGCCCCGGTGGACCT | Hindlll |
| FkbD-MLR | TTAATCTAGACGTCGCCTCGTCGTCGCT | Xbal |
| FkbD-MRF | TATATCTAGAGACACCGAAGGCGCGCTC | Xbal |
| FkbD-MRR | TTAAGAATTCGAACACCGAGGCCGTCCA | EcoRI |
| TcsDLF | GCTAAGCTTCTCAGGCGTCTGCGGATGC | Hindlll |
| TcsDLR | ATCGGATCCTTCGCTCACCGGGGCTGCC | BamHI |
| TcsDRF | AGCAGATCTGGCATGTTCTGGTCAGTCC | Bgll |
| TcsDRR | GTCGAATTCCATGCCACGAACGGGTCGA | EcoRI |
| FkbLLF | AATAAGCTTCCACGAGCCCGGT | Hindlll |
| FkbLLR | AAATCTAGACACATCGCGTTCGAC | Xbal |
| FkbLRF | AATTCTAGACACGGAGAGGATCTG | Xbal |
| FkbLRR | AAAGAATTCCCACCACCCCCG | EcoRI |

9-Deoxo-36,37-dihydro-prolyl FK506 was produced by culturing the prepared production strain, *Streptomyces kanamyceticus* ΔfkbD,tcsD,fkbL (Accession No. KCTC14171BP). A detailed description is as follows. To a 250 mL baffled flask, 50 mL of R2YE medium (103 g/L sucrose, 10 g/L glucose, 0.25 g/L potassium sulfate, 10.12 g/L magnesium chloride hexahydrate, 0.1 g/L casamino acid, 50 mL/L yeast extract (10%), 100 mL/L TES buffer (5.73%, pH 7.2), 10 mL/L potassium phosphate (0.5%), 80 mL/L calcium chloride dihydrate (3.68%), 15 mL/L L-proline (20%), 2 mL/L trace element solution, and 5 mL/L sodium hydroxide (1 N)) was added, and the production strain was seeded thereto, followed by pre-culture in an orbital shaker under conditions of 28°C and 180 rpm for two days. Next, 10 mL of the culture obtained by pre-culture for two days was seeded in a 3 L Erlenmeyer flask containing 1 L of R2YE medium. After seeding, incubation was performed under conditions of 28°C and 180 rpm for six days. After six days of the culture, 9-deoxo-36,37-dihydro-prolyl FK506 produced by a primary recovery process was extracted.

The primary recovery process was performed as follows. First, the equal amount of methanol was added to the culture medium, which was mixed for 30 minutes and centrifuged to remove cells. The extract from which the cells were removed was concentrated using a rotary evaporator. Then, the concentrated extract was dissolved in water, a double volume of ethyl acetate was added, mixed well, and then left until phase-separation occurred. When the phase-separation occurred, the upper organic solvent layer was recovered, and concentrated using a rotary evaporator, and the weight after concentration was measured. The extract obtained after the primary recovery process was passed through a column packed with silica gel. At this time, the amount of silica gel was 15 times the weight of the extract of the primary recovery process, and methanol and methylene chloride at 5 different ratios (fraction 1. 0:100, fraction 2. 1:100, fraction 3. 1:10, fraction 4. 1:1, fraction 5. 100:0) was used as a mobile phase. In fraction 3, 9-deoxo-36,37-dihydro-prolyl FK506 was identified. The fraction 3 thus obtained was concentrated using a rotary evaporator and finally purified using HPLC.

This product was freeze-dried to obtain 9-deoxo-36,37-dihydro-prolyl FK506 represented by Formula 1 in a powder form.

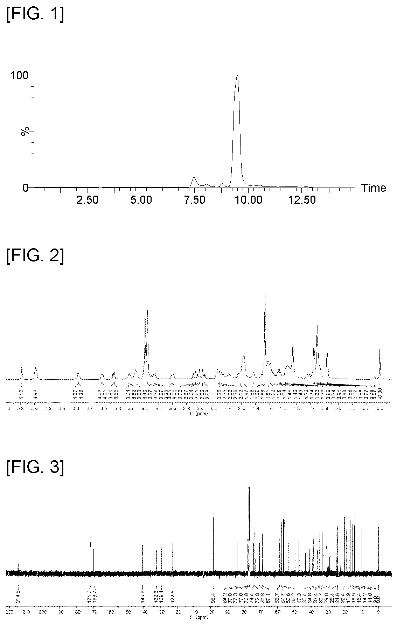

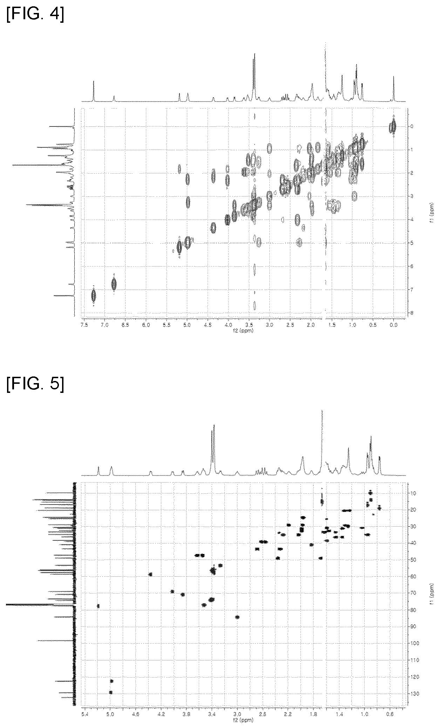

Identification of the prepared 9-deoxo-36,37-dihydro-prolyl FK506 was performed as follows. In detail, high-performance liquid chromatography analysis, mass spectrometry, and nuclear magnetic resonance analysis were performed. The results of analyzing 9-deoxo-36,37-dihydro-prolyl FK506 are summarized in Table 3 and FIGS. 1 to 6, and these results confirmed that 9-deoxo-36,37-dihydro-prolyl FK506 was produced from the prepared production strain *Streptomyces kanamyceticus* ΔfkbD,tcsD,fkbL.

The results of analyzing 9-deoxo-36,37-dihydro-prolyl FK506 (molecular formula: C₄₃H₇₁NO₁₁, molecular weight: 777.50) are shown in Table 3 below.

**[Table 3]**

| Analysis method | Analysis results | | |
|---|---|---|---|
| Mass spectrometry | (ESI-HR-MS) Calcd. for C₄₃H₇₁NNaO₁₁⁺: 800.4919, found: m/z 800.4924 | | |
| Nuclear magnetic resonance analysis | No. | Carbon (ppm) | Proton (ppm) |
| | 1 | 169.7 | |
| | 2 | 58.7 | 4.36 (1H, brd, *J=5.0* Hz) |
| | 3 | 20.0 | 1.97 (1H, m) 2.19 (1H, m) |
| | 4 | 24.6 | 1.97 (1H, m) 1.98 (1H, m) |
| | 5 | 47.3 | 3.53 (1H, m) 3.63 (1H, m) |
| | 6 | | |
| | 7 | | |
| | 8 | 171.6 | |
| | 9 | 39.0 | 2.55 (1H, d, *J=15.0* Hz) 2.62 (1H, d, *J=15.0* Hz) |
| | 10 | 98.4 | |
| | 11 | 38.4 | 1.59 (1H, m) |
| | 12 | 32.5 | 1.56 (1H, m) 1.98 (1H, m) |
| | 13 | 74.4 | 3.40 (1H, m) |
| | 14 | 70.8 | 3.85 (1H, brd, *J=10.0* Hz) |
| | 15 | 77.4 | 3.53 (1H, m) |
| | 16 | 36.3 | 1.34 (1H, m) 1.45 (1H, m) |
| | 17 | 25.4 | 1.60 (1H, m) |
| | 18 | 49.0 | 1.67 (1H, m) |
| | | | 2.36 (1H, m) |
| | 19 | 140.6 | |
| | 20 | 122.6 | 4.98 (1H, d, J=5.0 Hz) |
| | 21 | 53.4 | 3.26 (1H, m) |
| | 22 | 214.8 | |
| | 23 | 43.4 | 2.31 (1H, brd *J=15.0* Hz) |
| | | | 2.68 (1H, brd *J=15.0* Hz) |
| | 24 | 69.1 | 4.02 (1H, m) |
| | 25 | 40.9 | 1.82 (1H, m) |
| | 26 | 77.8 | 5.18 (1H, brs) |
| | 27 | 132.3 | |
| | 28 | 129.4 | 4.97 (1H, d, *J*=5.0 Hz) |
| | 29 | 34.8 | 2.26 (1H, m) |
| | 30 | 34.8 | 0.94 (1H, m) |
| | | | 2.04 (1H, m) |
| | 31 | 84.2 | 3.00 (1H, m) |
| | 32 | 73.6 | 3.40 (1H, m) |
| | 33 | 31.2 | 1.33 (1H, m) |
| | | | 1.98 (1H, m) |
| | 34 | 30.7 | 1.03 (1H, m) |
| | | | 1.59 (1H, m) |
| | 35 | 33.4 | 1.46 (1H, m) |
| | | | 1.63 (1H, m) |
| | 36 | 20.4 | 1.22 (2H, m) |
| | 37 | 14.0 | 0.88 (3H, t, *J*=7.5 Hz) |
| | 38 | 16.9 | 0.95 (3H, d, *J*=6.5 Hz) |
| | 39 | 18.9 | 0.76 (3H, d, *J*=6.5 Hz) |
| | 40 | 15.4 | 1.63 (3H, s) |
| | 41 | 9.8 | 0.85 (3H, d, *J*=6.5 Hz) |
| | 42 | 14.2 | 1.65 (3H, s) |
| | 43 | 56.2 | 3.36 (3H, s) |
| | 44 | 57.7 | 3.37 (3H, s) |
| | 45 | 56.6 | 3.40 (3H, s) |

From ¹H- and ¹³C-NMR, as characteristic functional groups, one ketone carbon (*δ*C 214.8) and two carbonyl carbons (*δ*C 171.6, 169.7), and two olefin backbones (*δ*C 140.6, 122.6; *δ*C 132.3, 129.4) were identified, and dioxygenated quaternary carbon (*δ*C 98.4), seven oxygenated methine carbons (*δ*C 84.2, 77.8, 77.4, 74.4, 73.6, 70.8, 69.1), and three methoxy carbons (*δ*C 57.7, 56.6, 56.2) were observed, six methyl carbons (*δ*C 18.9, 16.9, 15.4, 14.0, 9.8) were observed, and the compound was observed as a 43-carbon FK506 derivative.

To identify the exact structure, 2D NMR was examined. Proton linkages were examined from gCOSY, and as a result, coupling between H-2 and H-4 confirmed that the present compound has a prolyl backbone without CH₂ functional group, not FK506 having a pipecolyl backbone. The correlation of H-9(*δ*H 2.55, 2.62) with C-8(*δ*C 171.6), and C-10(*δ*C 98.4) from gHMBC data indicates that the present compound is a backbone in which C-9 is reduced with not ketone but CH₂. Exomethylene between C-36-C-37 which is a FK506 backbone was not observed, and H37 observed as a triplet in gCOSY 2D NMR showed coupling correlation between H36a/b and H36a/b and H35a/b, respectively, indicating dihydrogenation of the C-36-C-37 backbone. In addition, the presence of three methoxy functional groups at C-13, C-15, and C-31 was confirmed. Taken together, the present compound was identified as 9-deoxo-36,37-dihydro-prolyl FK506.

### Example 2: Preparation of 9-deoxo-31-O-demethyl-36,37-dihydro-prolyl FK506

In *Streptomyces kanamyceticus,* which is a strain producing FK506, inactivation of *fkbD-fkbM, tcsD,* and *fkbL* genes was induced using an in-frame deletion method by double cross-over homologous recombination according to a method described in Ban, Y. H. et al. (J. Nat. Prod. 2013, 76, 1091-1098) to prepare a *Streptomyces kanamyceticus* ΔfkbD-fkbM,tcsD,fkbL (Accession No. KCTC14170BP), which is a strain producing 9-deoxo-31-O-demethyl-36,37-dihydro-prolyl FK506.

In detail, to prepare a mutant, in which *fkbD-fkbM, tcsD,* and *fkbL* genes were deleted in *Streptomyces kanamyceticus* strain producing FK506, each gene was cloned into a pKC1139 vector and transferred to *Escherichia coli* ET12567/pUZ8002, and transformed to FK506-producing *Streptomyces kanamyceticus* strain via conjugation.

The method of preparing the strain may be explained in more detail by construction of an in-frame gene deletion plasmid and construction of a gene deletion strain.

In the construction of the in-frame gene deletion plasmid, an *E. coli-Streptomyces* shuttle vector pKC1139 was used for in-frame gene deletion. The plasmid construction was performed by PCR amplification of left- and right-flanking fragments of the target gene for deletion from fosmid DNA derived from *Streptomyces kanamyceticus.* Primer pairs FkbD-MLF/FkbD-MLR and FkbD-MRF/FkbD-MRR for the left-flanking fragments and the right-flanking fragments respectively were designed for deletion of the *fkbD-fkbM* gene. Primer pairs TcsDLF/TcsDLR and TcsDRF/TcsDRR for the left-flanking fragments and the right-flanking fragments respectively were designed for deletion of the *tcsD* gene. Primer pairs FkbLLF/FkbLLR and FkbLRF/FkbLRR for the left-flanking fragments and the right-flanking fragments respectively were designed for deletion of the *fkbL* gene. All the PCR fragments were isolated and digested with HindIII-Xbal or Xbal-EcoRl, and then cloned into the pKC1139 vector. Information regarding the strains, plasmids, and primers used in the present Example are shown in Tables 1 and 2.

The plasmids used for the construction of the gene deletion strain are summarized in Table 1. pΔfkbD-fkbM which is a plasmid to remove both C9 hydroxylase and 31-O-methyltransferase was transferred to *E. coli* ET12567/pUZ8002, and then introduced into *Streptomyces kanamyceticus* via conjugation, and the target genes were deleted by homologous recombination. A strain in which a single crossover between the deletion plasmid and the *Streptomyces kanamyceticus* chromosome had occurred was selected by cultivation of an apramycin-resistant transconjugant at 37°C (the non-permissive temperature for the pSG5-based replicon) in the presence of apramycin. Then, the obtained colony was subjected to three rounds of propagation in the absence of selection at 28°C to allow for the second crossover.

The two desired double crossover mutant, *i.e*., ΔfkbD-fkbM, was selected by the apramycin-sensitive phenotype, then verified by PCR, and selectively confirmed by Southern blot analysis.

To the prepared *fkbD-fkbM* gene-deleted *Streptomyces kanamyceticus* ΔfkbD-fkbM, pΔtcsD which is a plasmid for C21 side chain alteration was introduced, and the *tcsD* gene was deleted in the same manner as in the method of deleting the *fkbD-fkbM* gene. ΔfkbD-fkbM,tcsD was selected by apramycin-sensitive phenotype, then verified by PCR, and selectively confirmed by Southern blot analysis. Additionally, to the prepared *fkbD-fkbM* and tcsD-deleted *Streptomyces kanamyceticus* ΔfkbD-fkbM,tcsD, pΔfkbL, which is a plasmid for C1 prolyl ring formation, was introduced, and the *fkbL* gene was deleted in the same manner as in the methods of deleting the *fkbD-fkbM* and *tcsD* genes. ΔfkbD-fkbM,tcsD,fkbL was selected by apramycin-sensitive phenotype, then verified by PCR.

The prepared *Streptomyces kanamyceticus* ΔfkbD-fkbM,tcsD,fkbL which is *a fkbD-fkbM, tcsD, fkbL* gene-deleted strain was deposited at the Korean Collection for Type Cultures (KCTC) on April 14, 2020, under Accession No. KCTC14170BP.

9-Deoxo-31-*O-*demethyl-36,37-dihydro-prolyl FK506 was produced by culturing the prepared production strain, *Streptomyces kanamyceticus* ΔfkbD-fkbM,tcsD,fkbL (Accession No. KCTC14170BP). A detailed description is as follows. To a 250 mL baffled flask, 50 mL of R2YE medium (103 g/L sucrose, 10 g/L glucose, 0.25 g/L potassium sulfate, 10.12 g/L magnesium chloride hexahydrate, 0.1 g/L casamino acid, 50 mL/L yeast extract (10%), 100 mL/L TES buffer (5.73%, pH 7.2), 10 mL/L potassium phosphate (0.5%), 80 mL/L calcium chloride dihydrate (3.68%), 15 mL/L L-proline (20%), 2 mL/L trace element solution, and 5 mL/L sodium hydroxide (1 N)) was added, and the production strain was seeded thereto, followed by pre-culture in an orbital shaker under conditions of 28°C and 180 rpm for two days. Next, 10 mL of the culture obtained by pre-culture for two days was seeded in a 3 L Erlenmeyer flask containing 1 L of R2YE medium. After seeding, incubation was performed under conditions of 28°C and 180 rpm for six days. After six days of the culture, 9-deoxo-31-O-demethyl-36,37-dihydro-prolyl FK506 produced by a primary recovery process was extracted.

The primary recovery process was performed as follows. First, the equal amount of methanol was added to the culture medium, which was mixed for 30 minutes and centrifuged to remove cells. The extract from which the cells were removed was concentrated using a rotary evaporator. Then, the concentrated extract was dissolved in water, a double volume of ethyl acetate was added, mixed well, and then left until phase-separation occurred. When the phase-separation occurred, the upper organic solvent layer was recovered, and concentrated using a rotary evaporator, and the weight after concentration was measured. The extract obtained after the primary recovery process was passed through a column packed with silica gel. At this time, the amount of silica gel was 15 times the weight of the extract of the primary recovery process, and methanol and methylene chloride at 5 different ratios (fraction 1. 0:100, fraction 2. 1:100, fraction 3. 1:10, fraction 4. 1:1, fraction 5. 100:0) was used as a mobile phase. In fraction 3, 9-deoxo-31-O-demethyl-36,37-dihydro-prolyl FK506 was identified. The fraction 3 thus obtained was concentrated using a rotary evaporator and finally purified using HPLC.

This product was freeze-dried to obtain 9-deoxo-31-*O*-demethyl-36,37-dihydro-prolyl FK506 represented by Formula 2 in a powder form.

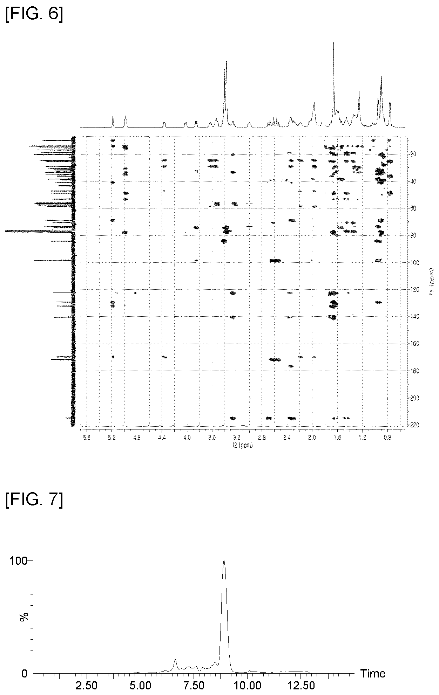

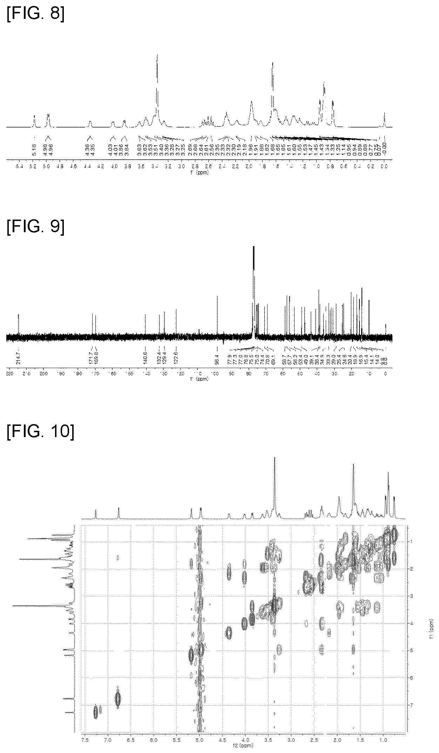

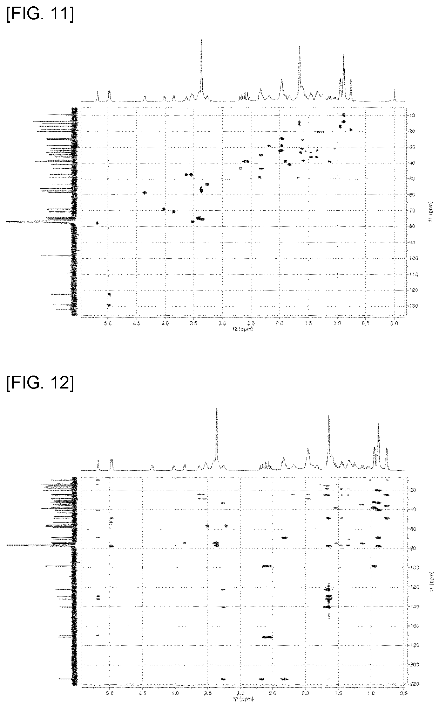

Identification of the prepared 9-deoxo-31-O-demethyl-36,37-dihydro-prolyl FK506 was performed as follows. In detail, high-performance liquid chromatography analysis, mass spectrometry, and nuclear magnetic resonance analysis were performed. The results of analyzing 9-deoxo-31-*O*-demethyl-36,37-dihydro-prolyl FK506 are summarized in Table 4 and FIGS. 7 to 12, and these results confirmed that 9-deoxo-31-O-demethyl-36,37-dihydro-prolyl FK506 was produced from the prepared production strain *Streptomyces kanamyceticus* ΔfkbD-fkbM,tcsD,fkbL.

The results of analyzing 9-deoxo-31-O-demethyl-36,37-dihydro-prolyl FK506 (molecular formula: C₄₂H₇₃NO₁₁, molecular weight: 763.49) are shown in Table 4 below.

**[Table 4]**

| Analysis method | Analysis results | | |
|---|---|---|---|
| Mass spectrometry | (ESI-HR-MS) Calcd. for C₄₂H₆₉NNaO₁₁⁺: 786.4763, found: m/z 786.4767 | | |
| Nuclear magnetic resonance analysis | No. | Carbon (ppm) | Proton (ppm) |
| | 1 | 169.8 | |
| | 2 | 58.7 | 4.36 (1H, brd, *J=5.0* Hz) |
| | 3 | 29.0 | 1.97 (1H, m) |
| | | | 2.19 (1H, m) |
| | 4 | 24.8 | 1.97 (1H, m) |
| | | | 1.98 (1H, m) |
| | 5 | 47.3 | 3.52 (1H, m) |
| | | | 3.63 (1H, m) |
| | 6 | | |
| | 7 | | |
| | 8 | 171.7 | |
| | 9 | 39.1 | 2.54 (1H, d, *J*=15.0 Hz) |
| | | | 2.63 (1H, d, *J=15.0* Hz) |
| | 10 | 98.4 | |
| | 11 | 38.4 | 1.59 (1H, m) |
| | 12 | 32.5 | 1.56 (1H, m) |
| | | | 1.98 (1H, m) |
| | 13 | 74.4 | 3.40 (1H, m) |
| | 14 | 70.8 | 3.85 (1H, brd, *J=10.0* Hz) |
| | 15 | 77.3 | 3.51 (1H, m) |
| | 16 | 36.3 | 1.34 (1H, m) |
| | | | 1.45 (1H, m) |
| | 17 | 25.4 | 1.60 (1H, m) |
| | 18 | 49.0 | 1.67 (1H, m) |
| | | | 2.35 (1H, m) |
| | 19 | 140.8 | |
| | 20 | 122.6 | 4.98 (1H, d, *J=5.0* Hz) |
| | 21 | 53.4 | 3.26 (1H, m) |
| | 22 | 216.3 | |
| | 23 | 43.5 | 2.31 (1H, brd *J=15.0* Hz) |
| | | | 2.68 (1H, brd *J=15.0* Hz) |
| | 24 | 69.1 | 4.02 (1H, m) |
| | 25 | 40.9 | 1.82 (1H, m) |
| | 26 | 77.9 | 5.18 (1H, brs) |
| | 27 | 132.4 | |
| | 28 | 129.4 | 4.97 (1H, d, |
| | | | *J=5.0* Hz) |
| | 29 | 34.9 | 2.32 (1H, m) |
| | 30 | 39.1 | 1.12 (1H, m) |
| | | | 1.90 (1H, m) |
| | 31 | 75.0 | 3.41 (1H, m) |
| | 32 | 75.5 | 3.34 (1H, m) |
| | 33 | 32.0 | 1.33 (1H, m) |
| | | | 1.95 (1H, m) |
| | 34 | 30.9 | 1.04 (1H, m) |
| | | | 1.61 (1H, m) |
| | 35 | 33.3 | 1.45 (1H, m) |
| | | | 1.63 (1H, m) |
| | 36 | 20.4 | 1.22 (2H, m) |
| | 37 | 14.0 | 0.88 (3H, t, *J*=7.5 Hz) |
| | 38 | 16.9 | 0.95 (3H, d, *J*=6.5 Hz) |
| | 39 | 18.9 | 0.76 (3H, d, *J*=6.5 Hz) |
| | 40 | 15.4 | 1.65 (3H, s) |
| | 41 | 9.8 | 0.89 (3H, d, *J*=6.5 Hz) |
| | 42 | 14.1 | 1.65 (3H, s) |
| | 43 | 57.7 | 3.36 (3H, s) |
| | 44 | 58.7 | 3.36 (3H, s) |

From ¹H- and ¹³C-NMR, as characteristic functional groups, one ketone carbon (*δ*C 216.3) and two carbonyl carbons (*δ*C 171.7, 169.8), and two olefin backbones (*δ*C 140.8, 122.6; *δ*C 132.4, 129.4) were identified, and dioxygenated quaternary carbon (*δ*C 98.4), seven oxygenated methine carbons (*δ*C 77.9, 77.3, 75.5, 75.0, 74.4, 70.8, 69.1), and two methoxy carbons (*δ*C 58.7, 57.7) were observed, five methyl carbons (*δ*C 18.9, 16.9, 15.4, 14.0, 9.8) were observed, and the compound was observed as a 42-carbon FK506 derivative. To identify the exact structure, 2D NMR was examined. Proton linkages were examined from gCOSY, and as a result, coupling between H-2 and H-4 confirmed that the present compound has a prolyl backbone. The correlation of H-9 (δH 2.54, 2.63) with C-8(*δ*C 171.7), C-10(*δ*C 98.4) from gHMBC data indicates that the present compound is a backbone in which C-9 is reduced with not ketone but CH₂. In addition, the compound has a structure in which two methoxy functional groups are linked to C-13 and C-15, and no methoxy is present at C-31.

Taken together, the present compound was identified as 9-deoxo-31-O-demethyl-36,37-dihydro-prolyl FK506.

### Example 3: Preparation of 9-deoxo-prolyl FK520

In *Streptomyces kanamyceticus,* which is a strain producing FK506, inactivation of *fkbD, tcsD,* and *fkbL* genes was induced using an in-frame deletion method by double cross-over homologous recombination according to a method described in Ban, Y. H. et al. (J. Nat. Prod. 2013, 76, 1091-1098) to prepare a *Streptomyces kanamyceticus* ΔfkbD,tcsD,fkbL (Accession No. KCTC14171BP), which is a strain producing 9-deoxo-prolyl FK520.

In detail, to prepare a mutant, in which *fkbD, tcsD,* and *fkbL* genes were deleted in *Streptomyces kanamyceticus* strain producing FK506, each gene was cloned into a pKC1139 vector and transferred to *Escherichia coli* ET12567/pUZ8002, and transformed to FK506-producing *Streptomyces kanamyceticus* strain via conjugation.

The method of preparing the strain may be explained in more detail by construction of an in-frame gene deletion plasmid and construction of a gene deletion strain.

In the construction of the in-frame gene deletion plasmid, an *E. coli-Streptomyces* shuttle vector pKC1139 was used for in-frame gene deletion. The plasmid construction was performed by PCR amplification of left- and right-flanking fragments of the target gene for deletion from fosmid DNA derived from *Streptomyces kanamyceticus.* Primer pairs FkbDLF/FkbDLR and FkbDRF/FkbDRR for the left-flanking fragments and the right-flanking fragments respectively were designed for deletion of the *fkbD* gene. Primer pairs TcsDLF/TcsDLR and TcsDRF/TcsDRR for the left-flanking fragments and the right-flanking fragments respectively were designed for deletion of the *tcsD* gene. Primer pairs FkbLLF/FkbLLR and FkbLRF/FkbLRR for the left-flanking fragments and the right-flanking fragments respectively were designed for deletion of the *fkbL* gene. All the PCR fragments were isolated and digested with HindIII-Xbal or Xbal-EcoRl, and then cloned into the pKC1139 vector. Information regarding the strains, plasmids, and primers used in the present Example are shown in Tables 1 and 2.

The plasmids used for the construction of the gene deletion strain are summarized in Table 1. pΔfkbD which is a plasmid to remove C9 hydroxylase was transferred to *E. coli* ET12567/pUZ8002, and then introduced into *Streptomyces kanamyceticus* via conjugation, and the target genes were deleted by homologous recombination. A strain in which a single crossover between deletion plasmid and the *Streptomyces kanamyceticus* chromosome had occurred was selected by cultivation of an apramycin-resistant transconjugant at 37°C (the non-permissive temperature for the pSG5-based replicon) in the presence of apramycin. Then, the obtained colony was then subjected to three rounds of propagation in the absence of selection at 28°C to allow for the second crossover. The two desired double crossover mutant, *i.e*., ΔfkbD, was selected by the apramycin-sensitive phenotype, then verified by PCR, and selectively confirmed by Southern blot analysis.

To the prepared *fkbD*-deleted *Streptomyces kanamyceticus* ΔfkbD, pΔtcsD which is a plasmid for C21 side chain alteration was introduced, and the *tcsD* gene was deleted in the same manner as in the method of deleting the *fkbD* gene. ΔfkbD,tcsD was selected by apramycin-sensitive phenotype, then verified by PCR, and selectively confirmed by Southern blot analysis. Additionally, to the prepared *fkbD-* and tcsD-deleted *Streptomyces kanamyceticus* ΔfkbD,tcsD, pΔfkbL, which is a plasmid for C1 prolyl ring formation, was introduced, and the fkbL gene was deleted in the same manner as in the methods of deleting the *fkbD* and *tcsD* genes. ΔfkbD,tcsD,fkbL was selected by apramycin-sensitive phenotype, then verified by PCR, and selectively confirmed by Southern blot analysis.

The prepared *Streptomyces kanamyceticus* ΔfkbD,tcsD,fkbL which is a *fkbD,tcsD,fkbL* gene-deleted strain was deposited at the Korean Collection for Type Cultures (KCTC) on April 14, 2020, under Accession No. KCTC14171BP.

9-Deoxo-prolyl-FK520 was produced by culturing the prepared production strain, *Streptomyces kanamyceticus* ΔfkbD,tcsD,fkbL (Accession No. KCTC14171BP). A detailed description is as follows. To a 250 mL baffled flask, 50 mL of R2YE medium (103 g/L sucrose, 10 g/L glucose, 0.25 g/L potassium sulfate, 10.12 g/L magnesium chloride hexahydrate, 0.1 g/L casamino acid, 50 mL/L yeast extract (10%), 100 mL/L TES buffer (5.73%, pH 7.2), 10 mL/L potassium phosphate (0.5%), 80 mL/L calcium chloride dihydrate (3.68%), 15 mL/L L-proline (20%), 2 mL/L trace element solution, and 5 mL/L sodium hydroxide (1 N)) was added, and the production strain was seeded thereto, followed by pre-culture in an orbital shaker under conditions of 28°C and 180 rpm for two days. Next, 10 mL of the culture obtained by pre-culture for two days was seeded in a 3 L Erlenmeyer flask containing 1 L of R2YE medium. After seeding, incubation was performed under conditions of 28°C and 180 rpm for six days. After six days of the culture, 9-deoxo-prolyl FK520 produced by a primary recovery process was extracted.

The primary recovery process was performed as follows. First, the equal amount of methanol was added to the culture medium, which was mixed for 30 minutes and centrifuged to remove cells. The extract from which the cells were removed was concentrated using a rotary evaporator. Then, the concentrated extract was dissolved in water, a double volume of ethyl acetate was added, mixed well, and then left until phase-separation occurred. When the phase-separation occurred, the upper organic solvent layer was recovered, and concentrated using a rotary evaporator, and the weight after concentration was measured. The extract obtained after the primary recovery process was passed through a column packed with silica gel. At this time, the amount of silica gel was 15 times the weight of the extract of the primary recovery process, and methanol and methylene chloride at 5 different ratios (fraction 1. 0:100, fraction 2. 1:100, fraction 3. 1:10, fraction 4. 1:1, fraction 5. 100:0) was used as a mobile phase. In fraction 3, 9-deoxo-prolyl FK520 was identified. The fraction 3 thus obtained was concentrated using a rotary evaporator and finally purified using HPLC.

This product was freeze-dried to obtain 9-deoxo-prolyl FK520 represented by Formula 3 in a powder form.

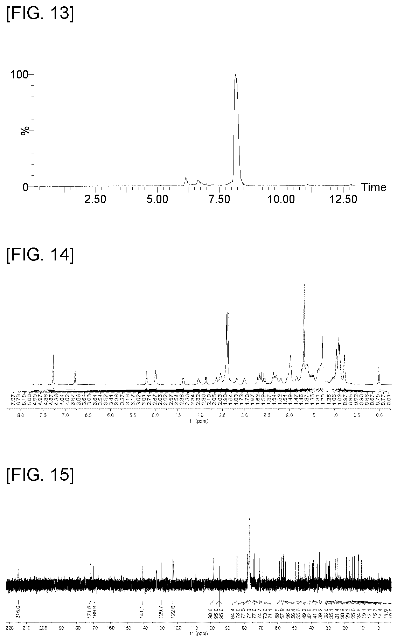

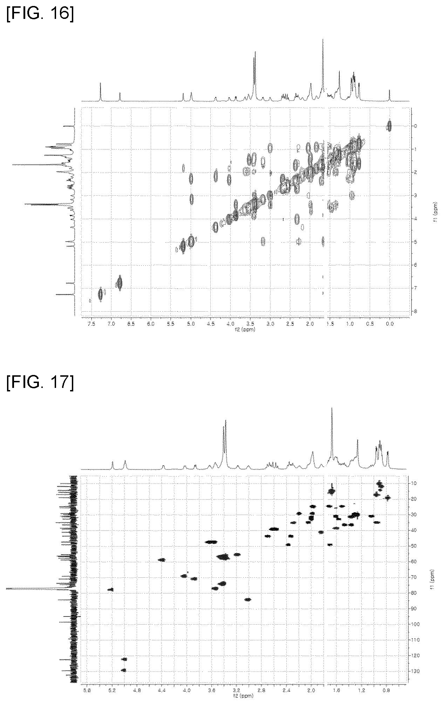

Identification of the prepared 9-deoxo-prolyl FK520 was performed as follows. In detail, high-performance liquid chromatography analysis, mass spectrometry, and nuclear magnetic resonance analysis were performed. The results of analyzing 9-deoxo-prolyl FK520 are summarized in Table 5 and FIGS. 13 to 18, and these results confirmed that 9-deoxo-prolyl FK520 was produced from the prepared production strain *Streptomyces kanamyceticus* ΔfkbD,tcsD,fkbL.

The results of analyzing 9-deoxo-prolyl FK520 (molecular formula: C₄₂H₆₉NO₁₁, molecular weight: 763.49) are shown in Table 5 below.

**[Table 5]**

| Analysis method | Analysis results | | |
|---|---|---|---|
| Mass spectrometry | (ESI-HR-MS) Calcd. for C₄₂H₆₉NNaO₁₁⁺: 786.4763, found: m/z 786.4768 | | |
| Nuclear magnetic resonance analysis | No. | Carbon (ppm) | Proton (ppm) |
| | 1 | 169.9 | |
| | 2 | 58.9 | 4.37 (1H, brd, *J=5.0* Hz) |
| | 3 | 29.2 | 1.98 (1H, m) |
| | | | 2.20 (1H, m) |
| | 4 | 25.8 | 1.97 (1H, m) |
| | | | 1.98 (1H, m) |
| | 5 | 47.5 | 3.56 (1H, m) |
| | | | 3.65 (1H, m) |
| | 6 | | |
| | 7 | | |
| | 8 | 171.8 | |
| | 9 | 39.2 | 2.57 (1H, d, *J=15.0* Hz) |
| | | | 2.62 (1H, d, *J=15.0* Hz) |
| | 10 | 98.6 | |
| | 11 | 38.6 | 1.59 (1H, m) |
| | 12 | 32.8 | 1.56 (1H, m) |
| | | | 1.99 (1H, m) |
| | 13 | 74.4 | 3.40 (1H, m) |
| | 14 | 71.1 | 3.85 (1H, brd, *J=10.0* Hz) |
| | 15 | 77.4 | 3.54 (1H, m) |
| | 16 | 36.3 | 1.34 (1H, m) |
| | | | 1.45 (1H, m) |
| | 17 | 25.4 | 1.61 (1H, m) |
| | 18 | 49.2 | 1.67 (1H, m) |
| | | | 2.36 (1H, m) |
| | 19 | 141.1 | |
| | 20 | 122.6 | 4.99 (1H, d, *J*=5.0 Hz) |
| | 21 | 55.5 | 3.18 (1H, m) |
| | 22 | 215.0 | |
| | 23 | 43.7 | 2.33 (1H, brd *J*=15.0 Hz), 2.68 (1H, brd *J*=15.0 Hz) |
| | 24 | 69.4 | 4.04 (1H, m) |
| | 25 | 41.2 | 1.83 (1H, m) |
| | 26 | 78.0 | 5.19 (1H, brs) |
| | 27 | 132.5 | |
| | 28 | 129.7 | 5.02 (1H, d, *J*=5.0 Hz) |
| | 29 | 35.1 | 2.28 (1H, m) |
| | 30 | 35.0 | 0.95 (1H, m), 2.05 (1H, m) |
| | 31 | 84.2 | 3.01 (1H, m) |
| | 32 | 73.8 | 3.42 (1H, m) |
| | 33 | 31.4 | 1.36 (1H, m) |
| | | | 1.96 (1H, m) |
| | 34 | 30.9 | 1.03 (1H, m) |
| | | | 1.60 (1H, m) |
| | 35 | 24.8 | 1.51 (1H, m) |
| | | | 1.71 (1H, m) |
| | 36 | 11.9 | 0.88 (3H, t, J=7.5 Hz) |
| | 37 | 17.1 | 0.96 (3H, d, *J*=6.5 Hz) |
| | 38 | 19.1 | 0.78 (3H, d, *J*=6.5 Hz) |
| | 39 | 15.7 | 1.67 (3H, s) |
| | 40 | 10.0 | 0.91 (3H, d, *J*=6.5 Hz) |
| | 41 | 14.4 | 1.67 (3H, s) |
| | 42 | 56.4 | 3.36 (3H, s) |
| | 43 | 57.9 | 3.37 (3H, s) |
| | 44 | 56.8 | 3.40 (3H, s) |

From ¹H- and ¹³C-NMR, as characteristic functional groups, one ketone carbon (*δ*C 215.0) and two carbonyl carbons (*δ*C 171.8, 169.9), and two olefin backbones (*δ*C 141.1, 122.6; *δ*C 132.5, 129.7) were identified, and dioxygenated quaternary carbon (*δ*C 98.6), seven oxygenated methine carbons (*δ*C 84.2, 78.0, 77.4, 74.4, 73.8, 71.1, 69.4), and three methoxy carbons (*δ*C 57.9, 57.9, 56.4) were observed, five methyl carbons (*δ*C 19.1, 17.1, 15.7, 14.4, 10.0) were observed, and the compound was observed as a 42-carbon FK506 derivative. To identify the exact structure, 2D NMR was examined. Proton linkages were examined from gCOSY, and as a result, coupling between H-2 and H-4 confirmed that the present compound has a prolyl backbone. The correlation of H-9(*δ*H 2.57, 2.62) with C-8(*δ*C 171.8), C-10(*δ*C 98.6) from gHMBC data indicates that the present compound is a backbone in which C-9 is reduced with not ketone but CH₂. In addition, the presence of three methoxy functional groups at C-13, C-15, and C-31 was confirmed. In addition, gCOSY coupling correlation and gHMBC long range correlation confirmed that the compound has a structure in which C-35 and C-36 are linked to C-21 through ethyl groups. Taken together, the present compound was identified as 9-deoxo-prolyl FK520.

### Example 4: Preparation of 9-deoxo-31-O-demethyl-prolyl FK520

In *Streptomyces kanamyceticus,* which is a strain producing FK506, inactivation of *fkbD-fkbM, tcsD,* and *fkbL* genes was induced using an in-frame deletion method by double cross-over homologous recombination according to a method described in Ban, Y. H. et al. (J. Nat. Prod. 2013, 76, 1091-1098) to prepare a *Streptomyces kanamyceticus* ΔfkbD-fkbM,tcsD,fkbL (Accession No. KCTC14170BP), which is a strain producing 9-deoxo-31-O-demethyl-prolyl FK520.

In detail, to prepare a mutant, in which *fkbD-fkbM, tcsD,* and *fkbL* genes were deleted in *Streptomyces kanamyceticus* strain producing FK506, each gene was cloned into a pKC1139 vector and transferred to *Escherichia coli* ET12567/pUZ8002, and transformed to FK506-producing *Streptomyces kanamyceticus* strain via conjugation.

The method of preparing the strain may be explained in more detail by construction of an in-frame gene deletion plasmid and construction of a gene deletion strain.

In the construction of the in-frame gene deletion plasmid, an *E. coli-Streptomyces* shuttle vector pKC1139 was used for in-frame gene deletion. The plasmid construction was performed by PCR amplification of left- and right-flanking fragments of the target gene for deletion from fosmid DNA derived from *Streptomyces kanamyceticus.* Primer pairs FkbD-MLF/FkbD-MLR and FkbD-MRF/FkbD-MRR for the left-flanking fragments and the right-flanking fragments respectively were designed for deletion of the *fkbD-fkbM* gene. Primer pairs TcsDLF/TcsDLR and TcsDRF/TcsDRR for the left-flanking fragments and the right-flanking fragments respectively were designed for deletion of the *tcsD* gene. Primer pairs FkbLLF/FkbLLR and FkbLRF/FkbLRR for the left-flanking fragments and the right-flanking fragments respectively were designed for deletion of the *fkbL* gene. All the PCR fragments were isolated and digested with HindIII-Xbal or Xbal-EcoRl, and then cloned into the pKC1139 vector. Information regarding the strains, plasmids, and primers used in the present Example are shown in Tables 1 and 2.

The plasmids used for the construction of the gene deletion strain are summarized in Table 1. pΔfkbD-fkbM which is a plasmid to remove both C9 hydroxylase and 31-O-methyltransferase was transferred to *E. coli* ET12567/pUZ8002, and then introduced into *Streptomyces kanamyceticus* via conjugation, and the target genes were deleted by homologous recombination. A strain in which a single crossover between the deletion plasmid and the *Streptomyces kanamyceticus* chromosome had occurred was selected by cultivation of an apramycin-resistant transconjugant at 37°C (the non-permissive temperature for the pSG5-based replicon) in the presence of apramycin. Then, the obtained colony was then subjected to three rounds of propagation in the absence of selection at 28°C to allow for the second crossover. The two desired double crossover mutant, i.e., ΔfkbD-fkbM, was selected by the apramycin-sensitive phenotype, then verified by PCR, and selectively confirmed by Southern blot analysis.

To the prepared *fkbD-fkbM* gene-deleted *Streptomyces kanamyceticus* ΔfkbD-fkbM, pΔtcsD which is a plasmid for C21 side chain alteration was introduced, and the *tcsD* gene was deleted in the same manner as in the method of deleting the *fkbD* gene. ΔfkbD-fkbM,tcsD was selected by apramycin-sensitive phenotype, then verified by PCR, and selectively confirmed by Southern blot analysis. Additionally, to the prepared *fkbD-fkbM* and *tcsD* gene-deleted *Streptomyces kanamyceticus* ΔfkbD-fkbM,tcsD, pΔfkbL, which is a plasmid for C1 prolyl ring formation, was introduced, and the *fkbL* gene was deleted in the same manner as in the methods of deleting the *fkbD-fkbM* and tcsD genes. ΔfkbD-fkbM,tcsD,fkbL was selected by apramycin-sensitive phenotype, then verified by PCR.

The prepared *Streptomyces kanamyceticus* ΔfkbD-fkbM,tcsD,fkbL which is *a fkbD-fkbM,tcsD,fkbL* gene-deleted strain was deposited at the Korean Collection for Type Cultures (KCTC) on April 14, 2020, under Accession No. KCTC14170BP.

9-Deoxo-31-*O*-demethyl-prolyl FK520 was produced by culturing the prepared production strain, *Streptomyces kanamyceticus* ΔfkbD-fkbM,tcsD,fkbL (Accession No. KCTC14170BP). A detailed description is as follows. To a 250 mL baffled flask, 50 mL of R2YE medium (103 g/L sucrose, 10 g/L glucose, 0.25 g/L potassium sulfate, 10.12 g/L magnesium chloride hexahydrate, 0.1 g/L casamino acid, 50 mL/L yeast extract (10%), 100 mL/L TES buffer (5.73%, pH 7.2), 10 mL/L potassium phosphate (0.5%), 80 mL/L calcium chloride dihydrate (3.68%), 15 mL/L L-proline (20%), 2 mL/L trace element solution, and 5 mL/L sodium hydroxide (1 N)) was added, and the production strain was seeded thereto, followed by pre-culture in an orbital shaker under conditions of 28°C and 180 rpm for two days. Next, 10 mL of the culture obtained by pre-culture for two days was seeded in a 3 L Erlenmeyer flask containing 1 L of R2YE medium. After seeding, incubation was performed under conditions of 28°C and 180 rpm for six days. After six days of the culture, 9-deoxo-31-O-demethyl-prolyl FK520 produced by a primary recovery process was extracted.

The primary recovery process was performed as follows. First, the equal amount of methanol was added to the culture medium, which was mixed for 30 minutes and centrifuged to remove cells. The extract from which the cells were removed was concentrated using a rotary evaporator. Then, the concentrated extract was dissolved in water, a double volume of ethyl acetate was added, mixed well, and then left until phase-separation occurred. When the phase-separation occurred, the upper organic solvent layer was recovered, and concentrated using a rotary evaporator, and the weight after concentration was measured. The extract obtained after the primary recovery process was passed through a column packed with silica gel. At this time, the amount of silica gel was 15 times the weight of the extract of the primary recovery process, and methanol and methylene chloride at 5 different ratios (fraction 1. 0:100, fraction 2. 1:100, fraction 3. 1:10, fraction 4. 1:1, fraction 5. 100:0) was used as a mobile phase. In fraction 3, 9-deoxo-31-O-demethyl-prolyl FK520 was identified. The fraction 3 thus obtained was concentrated using a rotary evaporator and finally purified using HPLC.

This product was freeze-dried to obtain 9-deoxo-31-O-demethyl-prolyl FK520 represented by Formula 4 in a powder form.

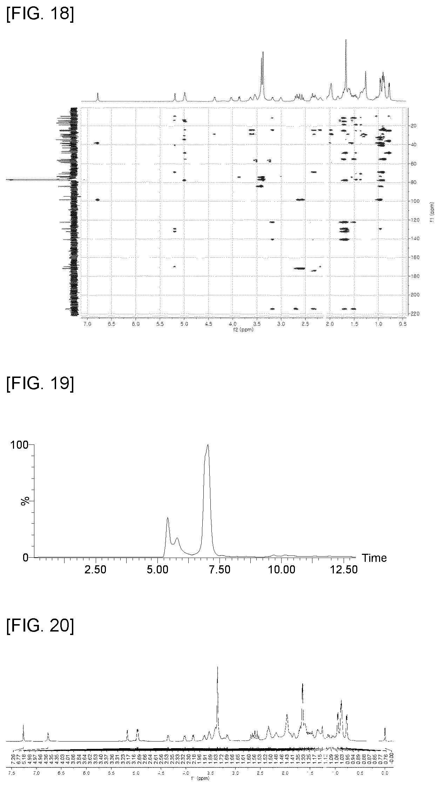

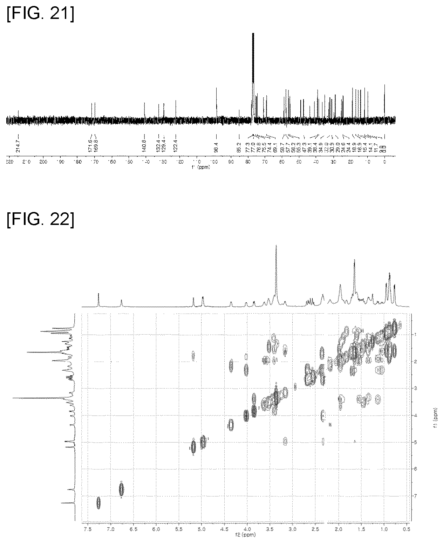

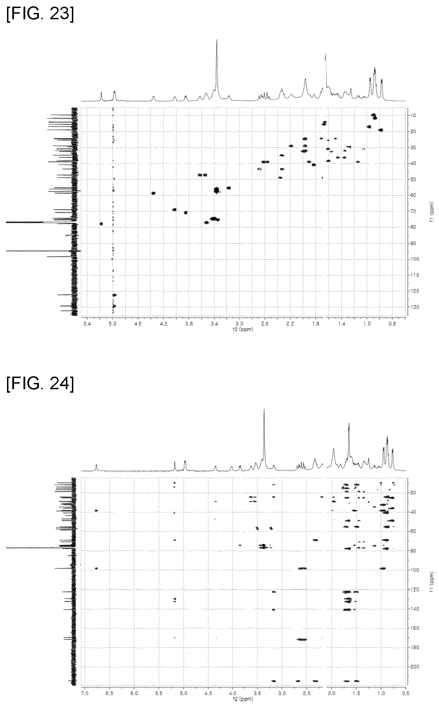

Identification of the prepared 9-deoxo-31-O-demethyl-prolyl FK520 was performed as follows. In detail, high-performance liquid chromatography analysis, mass spectrometry, and nuclear magnetic resonance analysis were performed. The results of analyzing 9-deoxo-31-O-demethyl-prolyl FK520 are summarized in Table 6 and FIGS. 19 to 24, and these results confirmed that 9-deoxo-31-O-demethyl-prolyl FK520 was produced from the prepared production strain *Streptomyces kanamyceticus* ΔfkbD-fkbM,tcsD,fkbL.

The results of analyzing 9-deoxo-31-O-demethyl-prolyl FK520 (molecular formula: C₄₁H₆₇NO₁₁, molecular weight: 749.97) are shown in Table 6 below.

**[Table 6]**

| Analysis method | Analysis results | | |
|---|---|---|---|
| Mass spectrometry | (ESI-HR-MS) Calcd. for C₄₁He₇NNaO₁₁⁺: 772.4614, found: *m*/*z* 772.4619 | | |
| Nuclear magnetic resonance analysis | No. | Carbon (ppm) | Proton (ppm) |
| | 1 | 169.8 | |
| | 2 | 58.7 | 4.36 (1H, brd, *J*=5.0 Hz) |
| | 3 | 29.0 | 1.96 (1H, m), 2.18 (1H, m) |
| | 4 | 25.4 | 1.97 (1H, m), 1.98 (1H, m) |
| | 5 | 47.3 | 3.54 (1H, m), 3.63 (1H, m) |
| | 6 | | |
| | 7 | | |
| | 8 | 171.6 | |
| | 9 | 39.1 | 2.56 (1H, d, *J*=15.0 Hz), 2.62 (1H, d, *J*=15.0 Hz) |
| | 10 | 98.4 | |
| | 11 | 38.4 | 1.59 (1H, m) |
| | 12 | 32.6 | 1.56 (1H, m), 1.98 (1H, m) |
| | 13 | 74.4 | 3.40 (1H, m) |
| | 14 | 70.9 | 3.85 (1H, brd, *J*=10.0 Hz) |
| | 15 | 77.3 | 3.52 (1H, m) |
| | 16 | 36.3 | 1.34 (1H, m), 1.45 (1H, m) |
| | 17 | 25.4 | 1.60 (1H, m) |
| | 18 | 49.0 | 1.69 (1H, m), 2.35 (1H, m) |
| | 19 | 140.8 | |
| | 20 | 122.4 | 4.97 (1H, d, *J*=5.0 Hz) |
| | 21 | 55.3 | 3.17 (1H, m) |
| | 22 | 214.7 | |
| | 23 | 43.8 | 2.32 (1H, brd *J*=15.0 Hz), 2.66 (1H, brd *J*=15.0 Hz) |
| | 24 | 69.1 | 4.02 (1H, m) |
| | 25 | 40.9 | 1.82 (1H, m) |
| | 26 | 77.9 | 5.18 (1H, brs) |
| | 27 | 132.4 | |
| | 28 | 129.4 | 4.97 (1H, d, *J*=5.0 Hz) |
| | 29 | 34.9 | 2.32 (1H, m) |
| | 30 | 39.1 | 1.12 (1H, m), 1.90 (1H, m) |
| | 31 | 75.0 | 3.41 (1H, m) |
| | 32 | 75.5 | 3.34 (1H, m) |
| | 33 | 32.0 | 1.33 (1H, m), 1.95 (1H, m) |
| | 34 | 30.9 | 1.04 (1H, m), 1.61 (1H, m) |
| | 35 | 24.6 | 1.49 (1H, m), 1.72 (1H, m) |
| | 36 | 11.7 | 0.87 (3H, t, *J*=7.5 Hz) |
| | 37 | 16.9 | 0.95 (3H, d, *J*=6.5 Hz) |
| | 38 | 18.9 | 0.77 (3H, d, *J*=6.5 Hz) |
| | 39 | 15.4 | 1.65 (3H, s) |
| | 40 | 9.8 | 0.89 (3H, d, *J*=6.5 Hz) |
| | 41 | 14.1 | 1.65 (3H, s) |
| | 42 | 56.2 | 3.37 (3H, s) |
| | 43 | 57.7 | 3.37 (3H, s) |

From ¹H- and ¹³C-NMR, as characteristic functional groups, one ketone carbon (*δ*C 214.7) and two carbonyl carbons (*δ*C 171.6, 169.8), and two olefin backbones (*δ*C 140.8, 122.4; *δ*C 132.4, 129.4) were identified, and dioxygenated quaternary carbon (*δ*C 98.4), seven oxygenated methine carbons (*δ*C 77.9, 77.3, 75.5, 75.0, 74.4, 70.9, 69.1), and two methoxy carbons (*δ*C 57.7, 56.2) were observed, five methyl carbons (*δ*C 18.9, 16.9, 15.4, 11.7, 9.8) were observed, and the compound was observed as a 41-carbon FK506 derivative with the reduced number of carbon. To identify the exact structure, 2D NMR was examined. Proton linkages were examined from gCOSY, and as a result, coupling between H-2 and H-4 confirmed that the present compound has a prolyl backbone, and coupling correlation of H21-H35-36 confirmed that the compound has the FK520 structure. The correlation of H-9(*δ*H 2.56, 2.62) with C-8(*δ*C 171.6), C-10(*δ*C 98.4) from gHMBC data indicates that the present compound is a backbone in which C-9 is reduced with not ketone but CH₂. In addition, two methoxy functional groups are linked to C-13 and C-15, indicating that the compound has a structure without methoxy at C-31. Taken together, the present compound was identified as 9-deoxo-31-O-demethyl-prolyl FK520.

### Example 5: Examination of immunosuppressive activity of four kinds of novel compounds

The reduced level of the immunosuppressive activity of the four kinds of novel compounds was examined using a common *in vitro* T-cell activation assay (J. Immunol. 143:718-726, 1989). The division of CD4+ T cells indicates that an immune response is taking place. When CD4+ T cells are stained with Cell Trace^{™} Violet (CTV), CTV retention of each cell decreases, as cells divide and T cells proliferate according to the immune response, and thus immunosuppressive activity was examined using the CTV retention as an index.

Single cells were isolated from the spleen of 6- to 8-week-old B6J laboratory mouse, and CD4+ T cells were isolated using MagniSort^{®} Mouse CD4 T cell Enrichment Kit (eBioscience). CD4+ T cells were stained with Cell Trace^{™} Violet (CTV) Cell Proliferation Kit (Molecular Probes) and FK506 or each of the four kinds of novel compounds was added at a concentration of 0.01 ng/mL, 0.1 ng/mL, 1 ng/mL, 10 ng/mL, 100 ng/mL, 1000 ng/mL, followed by incubation for 72 hours. For activation of T cells, Dynabeads^{®} Mouse T-Activator CD3/CD28 (Gibco) was used. As a control group, non-activated T cells were used. After incubation, CTV intensity was analyzed by flow cytometry.

Table 7 below and FIG. 25 show CTV intensity measured by flow cytometry, in which T cell proliferation and immunosuppressive activity of FK506 and four kinds of novel compounds were shown. As shown in Table 7 below and FIG. 25, all the novel compounds provided in the present invention showed remarkably reduced immunosuppressive activity, as compared with FK506. In particular, 9-deoxo-31-O-demethyl-prolyl FK520 showed the lowest immunosuppressive activity, among the FK506 derivatives which have been currently reported to maintain neuronal regeneration activity or to have improved neuronal regeneration activity.

**[Table 7]**

| Structural analogs | **Immunosuppression** IC₅₀ (ng/mL) |
|---|---|
| FK506 | 0.027 |
| 9-deoxo-36,37-dihydro-prolyl FK506 | 3088.1 |
| 9-deoxo-31-O-demethyl-36,37-dihydro-prolyl FK506 | 5556.7 |
| 9-deoxo-prolyl FK520 | 3288.8 |
| 9-deoxo-31-*O*-demethyl-prolyl FK520 | 7091.0 |

These results confirmed that the immunosuppressive activity of the four kinds of novel compounds according to the present invention was greatly reduced, as compared with FK506, and the four kinds of novel compounds showed IC₅₀ (ng/mL) of at least 1.14×10⁵-fold or more, indicating remarkably reduced immunosuppressive activity. Accordingly, it was considered that the pharmaceutical composition for preventing or treating neuronal diseases including one or more selected from the four kinds of novel compounds as an active ingredient may be used without a concern about side effects due to immunosuppressive activity.

### Example 6: Examination of neuronal growth-promoting activity of four kinds of novel compounds

The neuronal growth-promoting ability of the four kinds of novel compounds was examined using primary cultured murine hippocampal neurons according to a method reported by Jing Sun et al. (J. Neuroinflam. 15:180, 2018). In detail, primary cultured hippocampal neurons were treated with FK506 or one of the four kinds of novel compounds (treatment concentration: 1 ng/mL), and a control group did not receive any treatment. The neurite lengths were measured on photographic prints according to a previously reported method *(*J. Pharmacol. Exp. Ther. 302:1278-1285, 2002).

The results are shown in FIG. 26. As shown in FIG. 26, the four kinds of novel compounds of the present invention exhibited excellent neurite outgrowth effects.

In other words, it was confirmed that the four kinds of novel compounds of the present invention had excellent neuronal growth-promoting ability. In particular, it was confirmed that 9-deoxo-prolyl FK520 showed superior neurite outgrowth effect, as compared with FK506. Therefore, it could be concluded that the four kinds of novel compounds according to the present invention may be used for the purpose of preventing or treating neuronal diseases.

### Example 7: Examination of therapeutic effects of four kinds of novel compounds on neuronal diseases

The functional synaptogenic activity of the four kinds of novel compounds was examined using primary cultured murine hippocampal neurons according to a method reported by D. Park. et al. (Sci. Rep. 7:7260, 2017). In detail, hippocampal neurons on days 10-14 after primary culture while being exposed to FK506 or each of the four kinds of novel compounds (treatment concentration: 1 ng/mL) was subjected to patch-clamp recordings to measure frequency of excitatory synaptic currents. The frequency of excitatory synaptic currents is used as an index for changes in the number of synapses.

The measured frequency of excitatory synaptic currents is shown in FIG. 27.

As a result, 9-deoxo-36,37-dihydro-prolyl FK506 and 9-deoxo-31-O-demethyl-36,37-dihydro-prolyl FK506 showed increased spontaneous excitatory synaptic currents (sEPSCs) of 3.29±0.22 (p<0.001, n=7) and 2.63±0.27 (p<0.05, n=6), respectively, which are increased values, as compared with FK506 (2.61±0.18 (p<0.05, n=10)).

In other words, the frequency of excitatory synaptic currents was significantly increased in the group treated with 9-deoxo-36,37-dihydro-prolyl FK506 or 9-deoxo-31-O-demethyl-36,37-dihydro-prolyl FK506, among the four kinds of novel compounds of the present invention, indicating that the improvement of the neuronal growth-promoting activity observed in the groups treated with the novel compounds as in Example 6 may be associated with the increase of the functional synaptic formation.

### Example 8: Examination of therapeutic effects of four kinds of novel compounds on neurodegenerative diseases

The four kinds of novel compounds were examined for the density change of dopaminergic neuron fibers by the synthetic toxin MPTP. In detail, physiological saline, FK506, or the four kinds of novel compounds (treatment concentration: 5 mg/mL) were administered for 3 days using a cannula injection system capable of injecting drugs into specific brain regions of awake animals. On day 2, 1-methyl-4-phenyl-1,2,3,6-tetrahydropyridine (MPTP), which is a neurotoxic compound was injected intraperitoneally to create a Parkinson's disease animal model, and degeneration was induced in the nigrostriatal dopaminergic pathway associated with motor ability. On day 4, the number of dopaminergic neuronal cell bodies and the change in the density of neuron fibers were measured by performing immunohistochemistry (IHC) staining using TH antibody which is a dopaminergic neuron-specific marker.

The results of measuring the number of dopaminergic neuronal cell bodies and the change in the density of neuron fibers are shown in FIG. 28.

As a result, it was confirmed that the number of dopaminergic neuronal cell bodies was recovered, and the density of neuron fibers was recovered.

In other words, all the groups treated with each of the four kinds of novel compounds of the present invention, 9-deoxo-36,37-dihydro-prolyl FK506, 9-deoxo-31-*O*-demethyl-36,37-dihydro-prolyl FK506, 9-deoxo-prolyl FK520, and 9-deoxo-31-*O*-demethyl-prolyl FK520 showed superior recovery in the density of neuron fibers, as compared to those treated with FK506, indicating almost no immunosuppressive activity.

These results indicate that it is possible to use the four kinds of novel compounds for the purpose of treating neuronal diseases, particularly, as a drug material targeting neurodegenerative diseases.

### Example 9: Examination of safety of four kinds of novel compounds

To examine safety of the four kinds of novel compounds, an MTT assay for a cytotoxicity test, an Ames test for rapid evaluation of genotoxicity, and an hERG assay for evaluation of potential effects on cardiac repolarization were carried out. Methods generally used were conducted for the examination.

In detail, in the MTT assay, 80 to 800 times higher concentrations (100 nM to 1,000 nM) than the cytotoxicity test concentration (1 ng/mL=1.24 nM) were determined as test concentrations of the four kinds of novel compounds, and the drug cytotoxicity was measured in mammalian normal cells (HEK293 T Cell). In the Ames test, the four kinds of novel compounds were treated at a concentration of 4 µg, 16 µg, 64 µg per plate, respectively, and the four kinds of novel compounds were evaluated by examining whether reverse mutation was induced a histidine auxotroph strain (*Salmonella typhimurium* TA98) and a tryptophan auxotroph strain (*Escherichia coli* WP2 uvrA). In the hERG assay, 0 µM, 2 µM, 8 µM, and 32 µM of each of the four kinds of novel compounds were treated to two or more numbers of cells. One CHO hERG cell was perfused with a normal tyrode solution. After confirming that the hERG channel current was recorded consistently for 3 to 4 sweeps, an excipient control group and the groups, each treated with one of the four kinds of novel compounds, were perfused for about 1 minute to 2 minutes or longer, and the magnitude of the hERG channel current was allowed to constantly record, and the magnitude of the relative current (pA or nA) and the suppression rate (%) were measured.

Among them, the MTT assay results are shown in FIG. 29. As shown in FIG. 29, almost no cytotoxicity was observed in the four kinds of the novel compounds of the present invention.

In other words, no cytotoxicity was observed in the groups treated with each of the four kinds of the novel compounds of the present invention, 9-deoxo-36,37-dihydro-prolyl FK506, 9-deoxo-31-*O*-demethyl-36,37-dihydro-prolyl FK506, 9-deoxo-prolyl FK520, and 9-deoxo-31-*O*-demethyl-prolyl FK520, and they are safe materials without inducing reverse mutations in the test strains, as confirmed in the Ames test, and without risk of cardiac repolarization, as confirmed in the hERG assay.

Based on the above description, it will be understood by those skilled in the art that the present disclosure may be implemented in a different specific form without changing the technical characteristics thereof. In this regard, it should be understood that the above embodiment is not limitative, but illustrative in all aspects. The scope of the invention is defined by the appended claims.

### Effect of the invention

A pharmaceutical composition for preventing or treating neuronal diseases, the pharmaceutical composition including, as an active ingredient, any one or more selected from four kinds of novel compounds according to the present invention was confirmed to show remarkably reduced immunosuppressive activity while having excellent effects of neurite outgrowth and synaptogenic activity and therapeutic effects on neuronal diseases, in particular, neurodegenerative diseases in a Parkinson's disease mouse model induced by a neurotoxin MPTP, and also exhibiting high safety in cytotoxicity and safety tests. Accordingly, the four kinds of novel compounds of the present invention may be effectively used in the treatment of neuronal diseases without side effects due to immunosuppressive activity, and therefore, more fundamental therapeutic effects may be expected, as compared to existing drug therapy.

### [Accession No.]

Depositary Authority: Korea Research Institute of Bioscience and Biotechnology (KCTC)
Accession No.: KCTC14170BP
Date of deposit: 20200414
Depositary Authority: Korea Research Institute of Bioscience and Biotechnology (KCTC)
Accession No.: KCTC14171BP
Date of deposit: 20200414

## Claims

1. A compound selected from the group consisting of 9-deoxo-36,37-dihydro-prolyl FK506 represented by Formula 1, 9-deoxo-31-O-demethyl-36,37-dihydro-prolyl FK506 represented by Formula 2, 9-deoxo-prolyl FK520 represented by Formula 3, and 9-deoxo-31-O-demethyl-prolyl FK520 represented by Formula 4, or a pharmaceutically acceptable salt thereof:

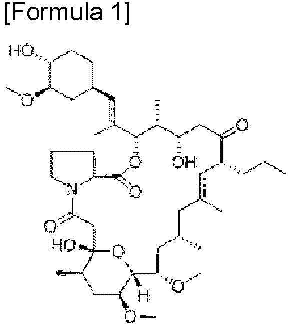

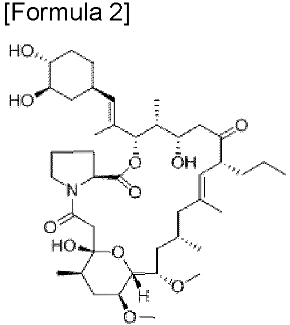

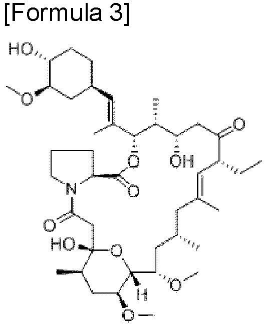

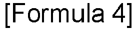

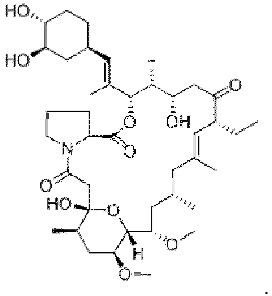

2. A compound according to claim 1 for use in the prevention or treatment of neuronal diseases.

3. The compound for use according to claim 2, wherein the neuronal diseases are any one or more selected from the group consisting of neuronal damage diseases, neurodegenerative diseases, peripheral nerve injury, traumatic brain injury, and cerebral infarction.

4. The compound for use according to claim 3, wherein the neuronal damage diseases are any one or more selected from the group consisting of epilepsy, stroke, cerebral infarction, ischemic encephalopathy, spinal cord injury disease, peripheral nerve disease, behavioral disorder, developmental disorder, mental retardation, Down syndrome, and schizophrenia.

5. The compound for use according to claim 3, wherein the neurodegenerative diseases are any one or more selected from the group consisting of dementia, Alzheimer's disease, Parkinson's disease, progressive supranuclear palsy, multiple system strophy, olivopontocerebellar atrophy (OPCA), Shy-Drager syndrome; striatonigral degeneration, Huntington's disease, amyotrophic lateral sclerosis (ALS), essential tremor, corticobasal ganglionic degeneration, diffuse Lewy body disease, Parkinson-ALS-dementia complex of Guam, and Pick's disease.

6. The compound for use according to claim 2, wherein the compound has reduced immunosuppressive activity.

7. The compound for use according to claim 2, wherein the compound exhibits neurite outgrowth and synaptogenic activity.

8. A pharmaceutical composition comprising the compound of claim 1 or a salt thereof, and an excipient.

9. A method of producing the compound of claim 1, which comprises:
(i) culturing *Streptomyces kanamyceticus* ΔfkbD,tcsD,fkbL (Accession No. KCTC14171BP) to produce 9-deoxo-36,37-dihydro-prolyl FK506;
(ii) culturing *Streptomyces kanamyceticus* ΔfkbD,tcsD,fkbL (Accession No. KCTC14171BP) to produce 9-deoxo-prolyl FK520;
(iii) culturing *Streptomyces kanamyceticus* ΔfkbD-fkbM,tcsD,fkbL (Accession No. KCTC14170BP) to produce 9-deoxo-31-*O*-demethyl-36,37-dihydro-prolyl FK506; or
(iv) culturing *Streptomyces kanamyceticus* ΔfkbD-fkbM,tcsD,fkbL (Accession No. KCTC14170BP) to produce 9-deoxo-31-*O*-demethyl-prolyl FK520.

## Patentansprüche

1. Verbindung, die aus der aus 9-Deoxo-36,37-dihydroprolyl FK506, das durch Formel 1 dargestellt ist, 9-Deoxo-31-O-demethyl-36,37-dihydroprolyl FK506, das durch Formel 2 dargestellt ist, 9-Deoxoprolyl FK520, das durch Formel 3 dargestellt ist, und 9-Deoxo-31-O-demethylprolyl FK520, das durch Formel 4 dargestellt ist, bestehenden Gruppe ausgewählt ist, oder ein pharmazeutisch annehmbares Salz davon:

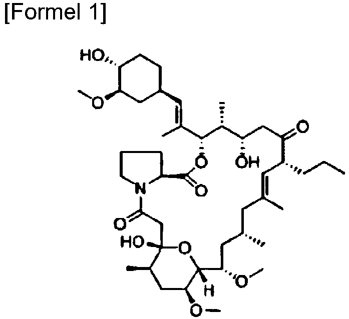

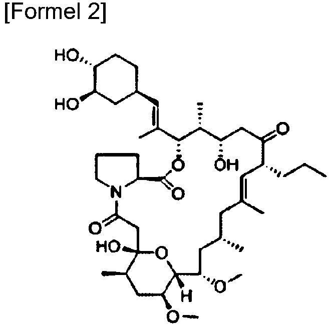

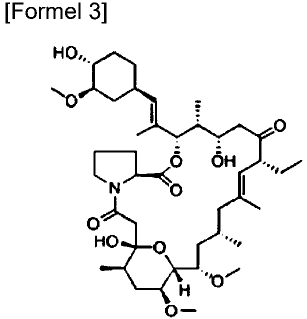

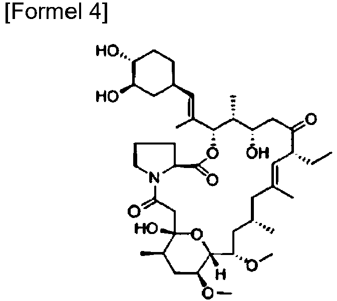

2. Verbindung nach Anspruch 1 zur Verwendung bei der Prävention oder Behandlung neuronaler Erkrankungen.

3. Verbindung zur Verwendung nach Anspruch 2, wobei die neuronalen Erkrankungen eine oder mehrere beliebige sind, die aus der aus neuronalen Schädigungserkrankungen, neurodegenerativen Erkrankungen, peripheren Nervenverletzungen, traumatischer Hirnverletzung und zerebralem Infarkt bestehenden Gruppe ausgewählt sind.

4. Verbindung zur Verwendung nach Anspruch 3, wobei die neuronalen Schädigungserkrankungen eine oder mehrere beliebige sind, die aus der aus Epilepsie, Schlaganfall, zerebralem Infarkt, ischämischer Enzephalopathie, Rückenmarksverletzungserkrankung, peripherer Nervenerkrankung, Verhaltensstörung, Entwicklungsstörung, geistiger Unterentwicklung, Down-Syndrom und Schizophrenie bestehenden Gruppe ausgewählt sind.

5. Verbindung zur Verwendung nach Anspruch 3, wobei die neurodegenerativen Erkrankungen eine oder mehrere beliebige sind, die aus der aus Demenz, Morbus Alzheimer, Morbus Parkinson, progressiver supranuklearer Lähmung, multipler Systematrophie, olivopontozerebellärer Atrophie (OPCA), Shy-Drager-Syndrom; striatonigraler Degeneration, Morbus Huntington, amyotropher Lateralsklerose (ALS), essentiellem Tremor, kortikobasaler Gangliondegeneration, diffuser Lewy-Körperchen-Erkrankung, dem Guam-Parkinson-ALS-Demenz-Komplex und Morbus Pick bestehenden Gruppe ausgewählt sind.

6. Verbindung zur Verwendung nach Anspruch 2, wobei die Verbindung eine reduzierte immunsuppressive Aktivität aufweist.

7. Verbindung zur Verwendung nach Anspruch 2, wobei die Verbindung eine Neuriten-Auswuchs- und synaptogene Aktivität aufweist.

8. Pharmazeutische Zusammensetzung, die eine Verbindung nach Anspruch 1 oder ein Salz davon und einen Hilfsstoff umfasst.

9. Verfahren zur Herstellung einer Verbindung nach Anspruch 1, das Folgendes umfasst:
(i) das Kultivieren von Streptomyces kanamyceticus ΔfkbD,tcsD,fkbL (Zugriffsnummer KCTC14171BP), um 9-Deoxo-36,37-dihydroprolyl FK506 herzustellen;
(ii) das Kultivieren von Streptomyces kanamyceticus ΔfkbD,tcsD,fkbL (Zugriffsnummer KCTC14171BP), um 9-Deoxoprolyl FK520 herzustellen;
(iii) das Kultivieren von Streptomyces kanamyceticus ΔfkbD-fkbM,tcsD,fkbL (Zugriffsnummer KCTC14170BP), um 9-Deoxo-31-O-demethyl-36,37-dihydroprolyl FK506 herzustellen; oder
(iv) das Kultivieren von Streptomyces kanamyceticus ΔfkbD-fkbM,tcsD,fkbL (Zugriffsnummer KCTC14170BP), um 9-Deoxo-31-O-demethylprolyl FK520 herzustellen.

## Revendications

1. Composé choisi dans le groupe comprenant le 9-désoxo-36,37-dihydro-prolyle FK506 représenté par la formule 1, le 9-désoxo-31-O-déméthyl-36,37-dihydro-prolyle FK506 représenté par la formule 2, le 9-désoxo-prolyle FK520 représenté par la formule 3 et le 9-désoxo-31-*O*-déméthyl-prolyle FK520 représenté par la formule 4, ou un sel pharmaceutiquement acceptable de celui-ci :

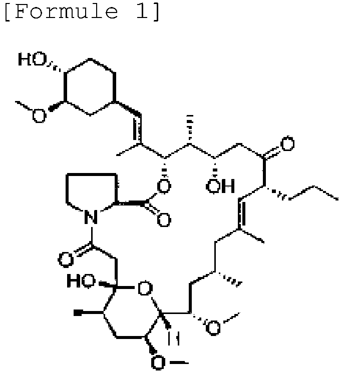

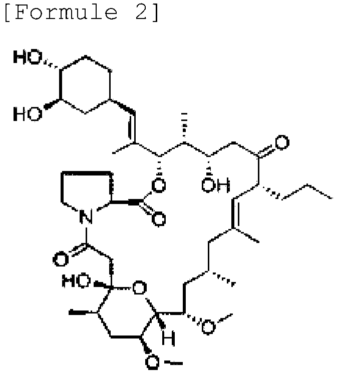

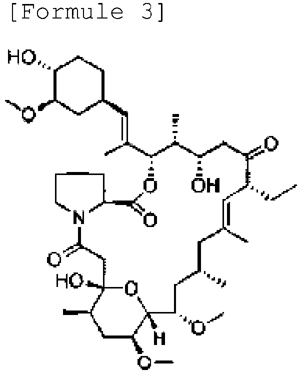

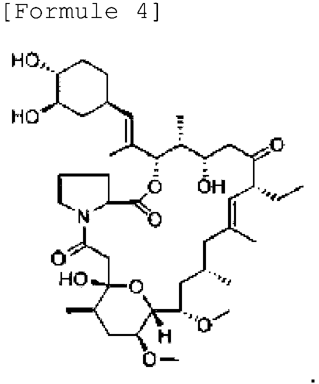

2. Composé selon la revendication 1 pour utilisation dans la prévention ou le traitement de maladies neuronales.

3. Composé pour utilisation selon la revendication 2, dans lequel les maladies neuronales sont une quelconque ou plusieurs choisies dans le groupe constitué de maladies à lésion neuronale, de maladies neurodégénératives, de lésions nerveuses périphériques, de lésions cérébrales traumatiques et d'infarctus cérébral.

4. Composé pour utilisation selon la revendication 3, dans lequel les maladies à lésions neuronales sont une ou plusieurs choisies dans le groupe comprenant l'épilepsie, un accident vasculaire cérébral, un infarctus cérébral, une encéphalopathie ischémique, une maladie de lésion de la moelle épinière, une maladie du nerf périphérique, un trouble du comportement, un trouble du développement, un retard mental, un syndrome de Down et la schizophrénie.

5. Composé pour utilisation selon la revendication 3, dans lequel les maladies neurodégénératives sont une quelconque ou plusieurs choisies dans le groupe comprenant la démence, la maladie d'Alzheimer, la maladie de Parkinson, la paralysie supranucléaire progressive, l'atrophie à systèmes multiples, l'atrophie olivo-ponto-cérébelleuse (OPCA), le syndrome de Shy-Drager ; la dégénérescence striato-nigrale, la maladie de Huntington, la sclérose latérale amyotrophique (SLA), le tremblement essentiel, la dégénérescence ganglionnaire corticobasale, la maladie à corps de Lewy diffus, le complexe Parkinson-ALS-démence de Guam et la maladie de Pick.

6. Composé pour utilisation selon la revendication 1, dans lequel le composé présente une activité immunosuppressive réduite.

7. Composé selon la revendication 1, dans lequel le composé présente une croissance des neurites et une activité synaptogénique.

8. Composition pharmaceutique comprenant le composé de la revendication 1 ou un sel de celui-ci, et un excipient.

9. Procédé de production du composé selon la revendication 1, qui comprend :
(i) la culture de *Streptomyces kanamyceticus* ΔfkbD,tcsD,fkbL (N° d'accession KCTC14171BP) pour produire du 9-désoxo-36,37-dihydro-prolyle FK506 ;
(ii) la culture de *Streptomyces kanamyceticus* ΔfkbD,tcsD,fkbL (N° d'accession KCTC14171BP) pour produire du 9-désoxo-prolyle FK520 ;
(iii) la culture de *Streptomyces kanamyceticus* ΔfkbD-fkbM,tcsD,fkbL (N° d'accession KCTC14170BP) pour produire du 9-désoxo-31-*O*-déméthyl-36,37-dihydro-prolyle FK506 ; ou
(iv) la culture de *Streptomyces kanamyceticus* ΔfkbD-fkbM,tcsD,fkbL (N° d'accession KCTC14170BP) pour produire du 9-désoxo-31-*O*-déméthyl-prolyle FK520.
